# EUROPEAN PATENT SPECIFICATION

(11) **EP 3 157 543 B1**
(45) Date of publication and mention of the grant of the patent: **12.11.2025**
(21) Application number: 15812073.3
(22) Date of filing: 23.06.2015
(51) Int. Cl.: G01N 33/564, C12N 9/22, C07K 5/00, C07K 7/00

(54) **COMPOSITIONS AND METHODS FOR THE DIAGNOSIS OF SYSTEMIC AUTOIMMUNE DISEASE**
ZUSAMMENSETZUNGEN UND VERFAHREN ZUR DIAGNOSE VON SYSTEMISCHER AUTOIMMUNERKRANKUNG
COMPOSITIONS ET PROCÉDÉS POUR DIAGNOSTIQUER UNE MALADIE AUTO-IMMUNE SYSTÉMIQUE

(30) Priority: 23.06.2014 US 201462016023 P
(43) Date of publication of application: 26.04.2017
(73) Proprietor: Inova Diagnostics, Inc., San Diego, CA 92131 (US)
(72) Inventor: MAHLER, Michael, San Diego, CA 92131 (US)
(74) Representative: Müller-Boré & Partner Patentanwälte PartG mbB
(86) International application number: PCT/US2015/037060
(87) International publication number: WO 2015/200242

(56) References cited:
- ANONYMOUS: "Rpp25 (C-16): sc-244048", SANTA CRUZ BIOTECHNOLOGY, 1 January 2012 (2012-01-01), pages 1 - 1, XP055375713, Retrieved from the Internet <URL:http://datasheets.scbt.com/sc-244048.pdf> [retrieved on 20170524]
- P. S. EDER ET AL: "Characterization of two scleroderma autoimmune antigens that copurify with human ribonuclease P", PROCEEDINGS NATIONAL ACADEMY OF SCIENCES PNAS, vol. 94, no. 4, 18 February 1997 (1997-02-18), US, pages 1101 - 1106, XP055451983, ISSN: 0027-8424, DOI: 10.1073/pnas.94.4.1101
- HANS VAN EENENNAAM ET AL: "Identity of the RNase MRP- and RNase P-associated Th/To autoantigen", ARTHRITIS & RHEUMATISM, vol. 46, no. 12, 1 December 2002 (2002-12-01), US, pages 3266 - 3272, XP055451759, ISSN: 0004-3591, DOI: 10.1002/art.10673
- M KUWANA ET AL: "Differences in autoantibody response to Th/To between systemic sclerosis and other autoimmune diseases", ANNALS OF THE RHEUMATIC DISEASES, 1 September 2002 (2002-09-01), England, pages 842 - 846, XP055451766, Retrieved from the Internet <URL:http://ard.bmj.com/content/61/9/842.full.pdf> [retrieved on 20180215], DOI: 10.1136/ard.61.9.842
- MICHAEL MAHLER ET AL: "Rpp25 is a major target of autoantibodies to the Th/To complex as measured by a novel chemiluminescent assay", ARTHRITIS RESEARCH AND THERAPY, BIOMED CENTRAL, LONDON, GB, vol. 15, no. 2, 12 April 2013 (2013-04-12), pages R50, XP021151852, ISSN: 1478-6354, DOI: 10.1186/AR4210
- SANTA CRUZ BIOTECHNOLOGY, INC., 1 June 2012 (2012-06-01), pages 1, XP055375713, Retrieved from the Internet <URL:http://datasheets.scbt.com/sc-244048.pdf> [retrieved on 20150906]
- "UniProtKB/ Swiss -Prot Suvmission Q9BUL9.", RPP25_HUMAN., 14 May 2014 (2014-05-14), XP055386353, Retrieved from the Internet <URL:http://www.uniprot.org/uniprot/Q9BUL9.txt?version=90> [retrieved on 20150907]

## Description

The present disclosure relates to the field of molecular biology and more specifically to methods for detecting antibodies in the serum of patients with systemic autoimmune rheumatic diseases. Specifically, the present invention relates to a purified peptide consisting of the amino acid sequence of SEQ ID NO: 25, a complex comprising said purified peptide and an anti-Th/To antibody, as well as methods for detecting an anti-Th/To antibody in a subject diagnosing systemic sclerosis (SSc) or limited cutaneous systemic sclerosis (lcSSc) in a human subject suspected of having SSc, determining the prognosis of systemic sclerosis (SSc) or limited cutaneous systemic sclerosis (IcSSc) in a human subject, or monitoring the efficacy of a systemic sclerosis (SSc) or limited cutaneous systemic sclerosis (IcSSc) treatment in a SSc or IcSSc patient, using said peptide, and a kit comprising the same.

Systemic autoimmune rheumatic diseases (SARD), including systemic sclerosis (SSc), are characterized by the presence of circulating autoantibodies to intracellular antigens. Mahler M et al, Ann N Y Acad Sci 1183:267-87 (2010); Mehra S et al, Autoimmun Rev 12:340- 54(2013). In SSc, the relevant autoantibodies include anti-topoisomerase I (topo-I, Scl-70) (See Mahler M et al, Autoimmun Rev 9:756-60(2010)), anti-centromere (CENP) (see Fritzler MJ et al, Autoimmun Rev 10:194-200(2011)), anti-RNA polymerase III (RNAP) antibodies (see Mahler M Ann N Y Acad Sci 1183:267-87 (2010);Van den Hoogen F, et al, Arthritis Rheum 65:2737- 47(2013)), as well as antibodies targeting Th To complex (see Ceribelli A et al, J Rheumatol 37:2071-5(2010); Van Eenennaam H et al, Arthritis Rheum 46:3266-72(2002); Van Eenennaam H et al, Clin Exp Immunol 130:532-40(2002); Okano Y et al, Arthritis Rheum 33: 1822-8(1990)), PM/Scl complex (exosome) (Mahler M et al, Autoimmun Rev 6:432-7(2007)) or U3RNP/fibrillarin (see Steen VD, Semin Arthritis Rheum 35:35-42(2005), Arnett FC et al, Arthritis Rheum 39: 1151-60(1996)). Several studies analyzed the epitope distribution on the autoantigens including CENP, PM/Scl (see Bluthner M et al, JMolMed (Berl) 78:47-54(2000)), RNA Pol III and Scl-70 (Mahler M et al, Ann N Y Acad Sci 1183:267-87 (2010)). In this context, Eder *et al.* (Eder, P. S. et al.; PNAS, 94(4); 1997; pp. 1101-1106) describes the characterization of two scleroderma autoimmune antigens that copurify with human ribonuclease P. However, very little is known about the epitope distribution on Th/To autoantigens. Thus, there remains a need to identify these epitopes of Th/To autoantigens in order to detect anti-Th To antibodies. This
application discloses for the first time linear epitopes on three of the Th/To autoantigens, namely Rpp25, Rpp38 and hPopl, which satisfies the need and provides related advantages.
The present invention is defined by the appended claims.

Described herein are compositions and methods for the diagnosis and prognosis of a disease related to an autoantibody against a subunit of the Th/To complex. The disease can be SSc. The subunit of the Th/To complex can be Rpp25, Rpp38, hPopI, or a combination thereof.

Described herein is a purified peptide having at least seven contiguous amino acids of an epitope derived from a subunit of a Th/To complex, wherein the epitope has an amino acid sequence selected from the group consisting of SEQ ID NOS: 14, 15, 21, 35, 36, 49, 53, 60-62. 65, 66, 71, 72, and 77-97 or a variant thereof. In one aspect, the epitope is derived from Rpp25 and has an amino acid sequence selected from the group consisting of SEQ ID NOS: 14, 15, 21, and 81-84; in another aspect, the epitope is derived from Rpp38 and has an amino acid sequence selected from the group consisting of SEQ ID NOS: 35, 36, 49, 53, and 85-92; in yet another aspect, the epitope is derived from hPopl and has an amino acid sequence selected from the group consisting of SEQ ID NOS: 60-62, 65, 66, 71, 72, 77-80, and 93-97.

In some aspects, the purified peptide has at least seven contiguous amino acids having at least 80%, at least 85%, at least 90%, at least 95%, at least 96%, at least 97%, at least 98%o, or at least 99% sequence identity with an amino acid sequence selected from the group consisting of SEQ ID NOS: 4-80 and 98-104. In some aspects, the purified peptide has an amino acid sequence selected from the group consisting of SEQ ID NOS: 4-80 and 98-104.

In one aspect, the purified peptide has the amino acid sequence of SEQ ID NO: 25 or SEQ ID NO: 102.

The purified peptide described herein can be a plurality of purified peptides. In some aspects, the plurality of peptides include a first purified peptide and a second purified peptide, wherein the first purified peptide having at least seven contiguous amino acids of a first epitope and the second purified peptide having at least seven contiguous amino acids of a second epitope, wherein the first epitope and the second epitope each is derived from Rpp25, Rpp38 or hPopl and has an amino acid sequence selected from the group consisting of SEQ ID NOS: 14, 15, 21, 35, 36, 49, 53, 60-62. 65, 66, 71, 72, and 77-97. In some aspects, the first epitope and
the second epitope are derived from the same subunit of the Th/To complex. In other aspects, the first epitope and the second epitope are derived from two different subunits of the Th/To complex.

The purified peptide described herein can also contain two or more epitope regions. In some aspects, the purified peptide has a first region having at least seven contiguous amino acids of a first epitope and a second region having at least seven contiguous amino acids of a second epitope, wherein the first epitope and the second epitope each is derived from Rpp25, Rpp38 or hPopl and has an amino acid sequence selected from the group consisting of SEQ ID NOS: 14, 15, 21, 35, 36, 49, 53, 60-62. 65, 66, 71, 72, and 77-97. In some aspects, the first epitope and the second epitope are derived from the same subunit of the Th/To complex. In other aspects, the first epitope and the second epitope are derived from two different subunits of the Th/To complex.

In some aspects, the purified peptide is a purified recombinant peptide encoded by cDNA. In some aspects, the purified peptide is a chemically synthesized.

Described herein is a complex including a purified peptide of this disclosure and one or more anti-Th/To antibodies. In some aspects, the complex is in solution. In some aspects, the complex is immobilized on a surface. In one aspect, the complex includes the purified peptide having the amino acid sequence of SEQ ID NO: 25 and one or more anti-Rpp38 antibodies. In another aspect, the complex includes the purified peptide having the amino acid sequence of SEQ ID NO: 102 and one or more anti- Rpp38 antibodies.

Also described herein is a method for detecting an anti-Th/To antibody in a subject including: a) contacting a sample from the subject with a purified peptide having at least seven contiguous amino acids of an epitope derived from a subunit of a Th/To complex, wherein the epitope has an amino acid sequence selected from the group consisting of SEQ ID NOS: 14, 15, 21, 35, 36, 49, 53, 60-62. 65, 66, 71, 72, and 77-97, or a variant thereof, to form a complex between an anti-Th/To antibody and the purified peptide, and b) detecting the presence or absence of the anti- Th/To antibody-purified peptide complex in the sample, wherein the anti-Th/To antibody can be an anti-Rpp25 antibody, an anti-Rpp38 antibody, an anti-hPopI antibody, or any combination thereof. In one aspect, the purified peptide has the amino acid sequence of SEQ ID NO: 25 or SEQ ID NO: 102.

In some aspects, detecting the presence or absence of the anti-Th/To antibody- peptide complex includes comparing the level of anti-Th/To antibody in the sample from the subject to a control level of anti-Th/To antibody in a sample from a healthy control individual, wherein an increase in the anti-Th/To antibody level in the sample compared to the control level indicates that the subject has SSc.

In some aspects, the method includes obtaining a sample from the subject. The subject can be suspected of having SSc, RA, pericarditis or interstitial lung disease. In some aspects, the subject has a negative ANA test.

Described herein is a method of diagnosing SSc in a human subject suspected of having SSc, including: a) contacting a sample from the subject with a purified peptide of the current disclosure to form a complex between an anti-Th/To antibody and the purified peptide, and b) detecting the presence or absence of the anti-Th/To antibody-purified peptide complex in the sample, wherein the presence of the anti-Th/To antibody-purified peptide complex in the sample indicates that the subject has SSc. In one aspect, the purified peptide has the amino acid sequence of SEQ ID NO: 25. In another aspect, the purified peptide has the amino acid sequence of SEQ ID NO: 102.

The anti-Th/To antibody can be an anti-Rpp25 antibody, an anti-Rpp38 antibody, an anti-hPopl antibody, or any combination thereof. In some aspects, the SSc is limited cutaneous SSc (lcSSc). In one aspect, the subject is positive for at least one autoantibody selected from the group consisting of an anti-topoisomerase I (topo-I) antibody, an anti-centromere (CENP) antibody and an anti-RNA polymerase III (RNAP) antibody. In yet another aspect, the subject is positive for an anti-PM/Scl complex (exosome) antibody or an anti-U3RNP/fibrillarin antibody.

In another aspect, the subject suspected of having SSc is negative for at least one autoantibody selected from the group consisting of an anti-topoisomerase I (topo-I) antibody, an anti-centromere (CENP) antibody and an anti-RNA polymerase III (RNAP) antibody. In yet another aspect, the subject suspected of having SSc is negative for an anti-PM/Scl complex (exosome) antibody or an anti-U3RNP/fibrillarin antibody.

Described herein is a method of determining the prognosis of SSc in a human subject, including: a) contacting a sample from the subject with the purified peptide of the current disclosure to form a complex between an anti-Th/To antibody and the purified peptide, and b) detecting the presence or absence of the anti-Th/To antibody-purified peptide complex in
the sample, wherein the presence of the anti-Th/To antibody-purified peptide complex in the sample indicates the course of SSc progression in the human subject. The anti-Th/To antibody can be an anti-Rpp25 antibody, an anti-Rpp38 antibody, an anti-hPopI antibody, or any combination thereof. In one aspect, the purified peptide has the amino acid sequence of SEQ ID NO: 25. In another aspect, the purified peptide has the amino acid sequence of SEQ ID NO: 102.

The human subject can be an asymptomatic subject suspected to be at risk of developing SSc, and the presence of the anti-Th/To antibody-purified peptide complex in the sample indicates that the subject is at a greater risk of developing SSc than the absence of the anti-Th/To antibody-purified peptide complex.

The human subject can also be a SSc patient having a clinic symptom of SSc. In some aspects, the presence of the anti-Th/To antibody-purified peptide complex in the sample predicts a more severe clinical course of SSc disease progression than the absence of the anti-Th/To antibody-purified peptide complex.

Described herein is a method of monitoring the efficacy of a SSc treatment in a SSc patient, including: a) contacting two or more samples obtained from the patient at a first and at least one subsequent time point during SSc treatment with the purified peptide of the current disclosure to form a complex between an anti-Th/To antibody and the purified peptide; b) determining the levels of the anti-Th/To antibody in the two or more samples; and c) comparing the levels of anti-Th/To antibodies in the two or more samples, wherein a decreased level of the anti-Th/To antibody in a sample obtained at a subsequent time point relative to a level of the anti-Th/To antibody in a sample obtained at the first time point indicates that the SSc treatment is efficacious. In one aspect, the purified peptide has the amino acid sequence of SEQ ID NO: 25. In another aspect, the purified peptide has the amino acid sequence of SEQ ID NO: 102. The anti-Th/To antibody can be an anti-Rpp25 antibody, an anti- Rpp38 antibody, an anti-hPopl antibody, or any combination thereof.

Also described herein is a kit for detecting an anti-Th/To antibody in a sample from a subject or for diagnosing an autoimmune disease, including a purified peptide of the current disclosure and an ancillary reagent, the anti-Th/To antibody can be an anti-Rpp25 antibody, an anti-Rpp38 antibody, an anti-hPopl antibody, or any combination thereof.

In one aspect, the purified peptide used in the kit has the amino acid sequence of SEQ ID NO: 25. In another aspect, the purified peptide has the amino acid sequence of SEQ ID NO: 102.

In some aspects, the ancillary reagent can be a secondary antibody, a detection reagent, an immobilization buffer, a blocking buffer, a washing buffer, and a detection buffer, or any combination thereof. In some aspects, the kit also includes a microtiter plate having wells. In some aspects, the purified peptide is immobilized in one or more wells of the microtiter plate.

In some aspects, the kit has a packaging having a label indicating that the kit is used for diagnosis, prognosis or monitoring of SSc, RA, ILD or IcSSc. In some aspects, the label indicates that the kit is used as an In Vitro Diagnostic companion diagnostic device. In some other aspects, the label indicates that the kit is used with a SSc drug. In some aspects, the label is FDA-approved. The figures show:
Figure 1: Epitope mapping of Rpp25, Rpp38 and hPopl using solid phase peptides. The peptide arrays stained with pools of Th/To positive samples (a, b) show several immunoreactive regions on all three proteins. Reactivity with the pool of samples derived from systemic lupus erythematosus (SLE) patients was significantly lower (c). The most pronounced reactivity was found on Rpp38 (indicated in the block). No significant reactivity was found on the array incubated using the pool of SLE patients samples.

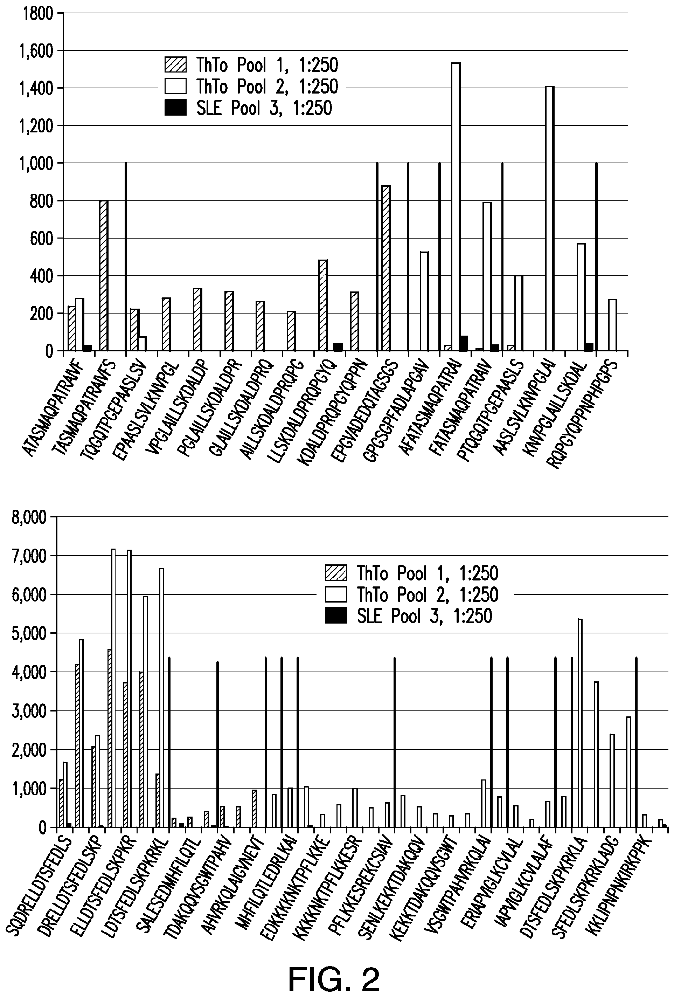

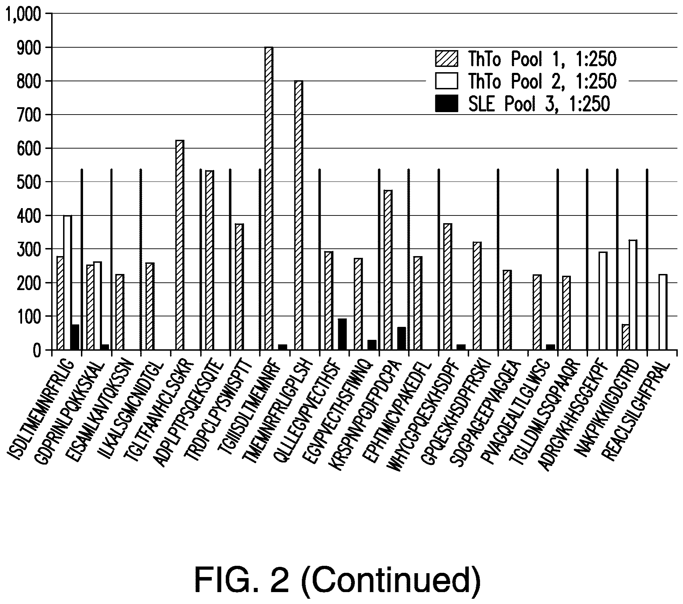

Figure 2: Peptides reactive with Th/To serum pools. Peptides that showed reactivity (>200 units) with at least one of the two Th/To serum pools, but not with the systemic lupus erythematosus pool (positive peptides) were selected and analyzed. Positive peptides derived from Rpp25 (SEQ ID NOS 4-21, respectively, in order of appearance) are shown in a), from Rpp38(SEQ ID NOS 22, 24, 26, 28, 30, 32, 34, 36, 38, 40, 42, 44, 46, 48, 50, 52, 54, 56 and 58, respectively, in order of appearance) in b) and from hPopl (SEQ ID NOS 60-80, respectively, in order of appearance) in c). Different epitope regions are separated by the vertical lines in the graphs.
Figure 3: Supervised cluster analysis. The supervised cluster analysis shows the autoantibody profile to Th/To derived peptides of the SSc samples with nucleolar pattern in a heat-map and their relation in a dendogram. Peptide 3, 6 and 8 showed the best correlation with the diagnosis.
Figure 4: Major linear epitope on Rpp38. The major linear epitope on Rpp38 is shown in a map a) and using the Rpp38 amino acid sequence (SEQ ID NO: 2) b).
Figure 5 : Antibodies to Rpp38 peptide 3 and Rpp38 peptide 6. Reactivity to the two peptides was clearly higher in SSc samples with nucleolar staining pattern compared to disease controls.
Figure 6: Correlation between anti-Rpp25 and anti-Rpp38 229-243 peptide (SEQ ID NO: 25) antibodies. The reactivity between anti-Rpp25 antibodies measured by BIO-FLASH and anti-Rpp38 peptide antibodies shows significant correlation.

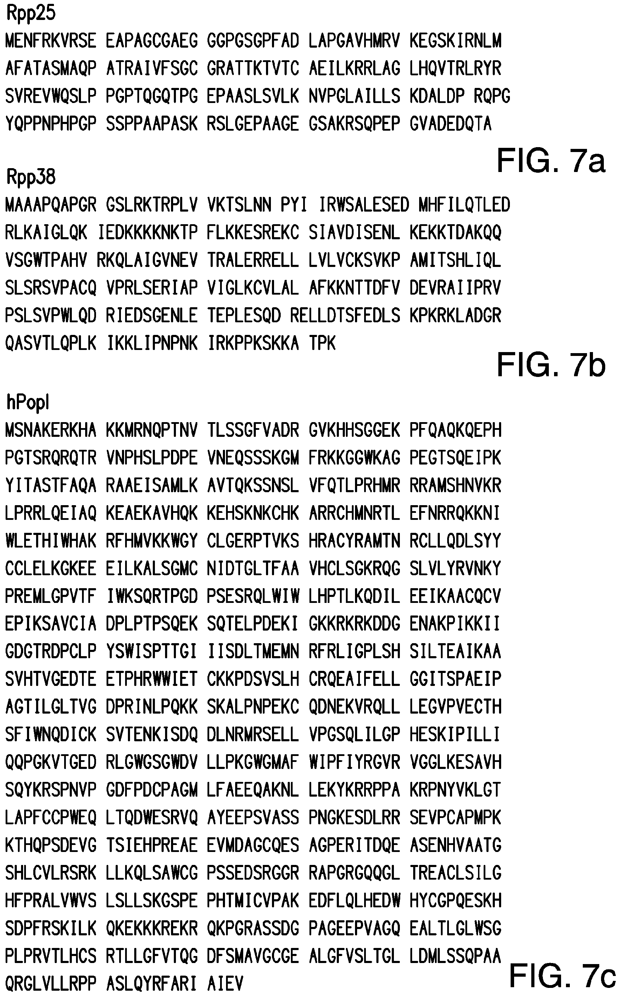

Figure 7: Exemplary amino acid sequences of the subunits of Th/To complexes. a). Rpp25(SEQ ID NO: 1); b) Rpp38(SEQ ID NO: 2); c) hPop1(SEQ ID NO: 3).

Systemic autoimmune rheumatic diseases (SARD), including systemic sclerosis (SSc), are characterized by the presence of circulating autoantibodies to intracellular antigens (Mahler M et al, Ann N Y Acad Sci 1183:267-87 (2010); Mehra S et al, Autoimmun Rev 12:340-54(2013)). Clinic symptoms of SSc include hardening and tightening of patches of skin, stiffness and tightness of skin of fingers, hands, and forearm, exaggerated responses to cold temperatures or emotional distress, which can cause numbness, pain or color changes in the fingers or toes (also known as Raynaud's phenomenon), hair loss, and acid reflux. Other clinical feature of SSc can include interstitial lung disease, vascular damage and fibrosis and muscle pain. In some cases, additional organs can be affected by the disease. In SSc the most relevant autoantibodies include anti-topoisomerase I (topo-I, Scl-70) (Mahler M et al, Autoimmun Rev 9:756-60(2010)), anti-centromere (CENP) (Fritzler MJ et al, Autoimmun Rev 10:194-200(2011)) and anti-RNA polymerase III (RNAP) antibodies which are also part of the recently revised classification criteria (Mahler M et al, Ann N YAcad Sci 1183:267-87 (2010); Van den Hoogen F et al, Arthritis Rheum 65:2737-47(2013)). Besides these, several other autoantibodies can be present in SSc patients including autoantibodies targeting the PM/Scl complex (also known as the exosome) (Mahler M et al, Autoimmun Rev 6:432-7(2007)), U3RNP/fibrillarin (Steen VD, Semin Arthritis Rheum 35:35-42(2005); Arnett FC et al, Arthritis Rheum 39:1151-60(1996)) and the Th/To autoantigens (Ceribelli A et al, J Rheumatol 37:2071-5(2010); Van Eenennaam H et al, Arthritis Rheum 46:3266-72(2002); Van Eenennaam H et al, Clin Exp Immunol 130:532-40(2002); Okano Y et al, Arthritis Rheum 33:1822-8(1990)). Anti-Th/To antibodies are one of the specificities or subtypes that reportedly show homogenous nucleolar staining in conventional indirect immunofluorescence (IIF) antinuclear antibody (ANA) tests (Ceribelli A et al, J Rheumatol 37:2071-5(2010); Fischer A et al, J Rheumatol 33:1600-5(2006); Wiik AS et al, J Autoimmun 35:276-90(2010)). In SSc, anti-Th/To antibodies have been associated with the lcSSc subset and the reported prevalence ranged from 1 to 13% (Ceribelli A et al, J Rheumatol 37:2071-5(2010); Graf SW et al, Int J Rheum Dis;15:102-9(2012*);* Mierau R et al, Arthritis Res Ther 13:R172(2011)). In addition to SSc, a few reports have described anti-Th/To antibodies in rheumatoid arthritis (RA) and interstitial lung disease (ILD) (Kuwana M et al, Ann Rheum Dis 61:842-6(2002); Koenig M et al, Arthritis Res Ther 9:R78(2007)).

The Th/To antigen complex is a multi-protein-RNA complex (human RNase MRP complex) consisting of a catalytic RNA and at least 10 proteins (Mehra S et al, Autoimmun Rev 12:340-54(2013); Van Eenennaam H et al, Arthritis Rheum 46:3266-72(2002)). RNase MRP is a ubiquitously expressed eukaryotic endoribonuclease that specifically cleaves various RNAs, including ribosomal, messenger, and mitochondrial RNAs (Van Eenennaam H et al, Arthritis Rheum 46:3266-72(2002)). Almost all protein subunits of the RNase MRP and the evolutionarily related RNase P complex have been reported as autoantibody targets in SARD patients (Van Eenennaam H et al, Arthritis Rheum 46:3266-72(2002); Van Eenennaam H et al, Clin Exp Immunol 130:532-40(2002); Kuwana M et al, Ann Rheum Dis 61:842-6(2002)). Rpp25 (Mahler M et al, Arthritis Res Ther 15:R50(2013)), Rpp38 (Kuwana M et al, Ann Rheum Dis 61:842-6(2002)) and hPop1 (Van Eenennaam H et al, Arthritis Rheum 46:3266-72(2002)) have been described as main autoantigens. Historically, anti-Th/To antibodies have been detected by immunoprecipitation (IP) (Ceribelli A et al, J Rheumatol 37:2071-5(2010)). While some studies tested serological cohorts, other investigations analyzed samples initially identified based on a nucleolar IIF staining pattern. Several years ago, commercial line immunoassays (LIA) for the detection of anti-Th/To antibodies based on the hPop1 target became available and were evaluated in two independent studies (Villalta D et al, Autoimmun Rev 12:114-20 (2012); Bonroy C et al, J Immunol Methods 379:53-60(2012)). In addition, an IP real-time PCR assay has been developed and evaluated (Ceribelli A et al, Arthritis Res Ther 14:R128(2012)). Just recently, a chemiluminescent immunoassay based on recombinant Rpp25 has been developed and evaluated (Mahler M et al, Arthritis Res Ther 15:R50(2013)).

Although known for over 20 years, the reported clinical association of anti-Th/To antibodies is inconsistent except their association with lcSSc. Furthermore, anti-Th/To antibodies are rarely used in routine testing algorithms to aid in the diagnosis and management of SSc patients due to unavailability of the IP assay or alternative methods. Antinuclear antibodies (ANA) are present in more than 90% of the patients and play an important role in the diagnosis of SSc. Lastly, very little is known about the epitope distribution of autoantibodies to the major Th/To antigens. The current application discloses epitope distribution on Rpp25, Rpp38 and hPop1 and provides peptides that can be used for the diagnosis and management of patients of SARD, including SSc.

The Th/To complex is a multi-protein-RNA complex (human RNase MRP complex) consisting of a catalytic RNA and at least 10 proteins. A "subunit of the Th/To complex" refers to a protein that constitutes the Th/To complex. A subunit of the Th/To complex can include the protein Rpp25, Rpp38 or hPop1. A subunit of the Th/To complex also can include two, three or more proteins that constitute the Th/To complex. For example, a subunit can include the proteins Rpp25 and Rpp38, Rpp25 and hPop1, Rpp38 and hPop1 as well as any combination or permutation of the proteins Rpp25, Rpp38 and/or hPop1, including all three proteins.

The term "ribonuclease P protein subunit p25" or "Rpp25" refers to a protein , including any native Rpp25 from any vertebrate source, including mammals such as human, and, can include related Rpp25 proteins such as Single Nucleotide Polymorphism (SNP) variants thereof. An exemplary amino acid sequence of Rpp25 is the sequence of human Rpp25 as provided below:

MENFRKVRSE EAPAGCGAEG GGPGSGPFAD LAPGAVHMRV KEGSKIRNLM
AFATASMAQP ATRAIVFSGC GRATTKTVTC AEILKRRLAG LHQVTRLRYR

SVREVWQSLP PGPTQGQTPG EPAASLSVLK NVPGLAILLS KDALDPRQPG
YQPPNPHPGP SSPPAAPASK RSLGEPAAGE GSAKRSQPEP GVADEDQTA (SEQ ID NO:
1)

The term "ribonuclease P protein subunit p38" or "Rpp38" refers to a protein, including any native Rpp38 from any vertebrate source, including mammals such as human, and, can include related Rpp38 proteins, such as SNP variants thereof. An exemplary amino acid sequence of Rpp38 is the sequence of human Rpp38 as provided below:

MAAAPQAPGR GSLRKTRPLV VKTSLNNPYI IRWSALESED MHFILQTLED
RLKAIGLQKI EDKKKKNKTP FLKKESREKC SIAVDISENL KEKKTDAKQQ
VSGWTPAHVR KQLAIGVNEV TRALERRELL LVLVCKSVKP AMITSHLIQL
SLSRSVPACQ VPRLSERIAP VIGLKCVLAL AFKKNTTDFV DEVRAIIPRV
PSLSVPWLQD RIEDSGENLE TEPLESQDRE LLDTSFEDLS KPKRKLADGR
QASVTLQPLK IKKLIPNPNK IRKPPKSKKA TPK (SEQ ID NO: 2)

The term "ribonucleases P/MRP protein subunit POP1" or "hPop1" refers to a protein, including any native hPop1 from any vertebrate source, including mammals such as human, and, can include related hPop1 proteins such as SNP variants thereof. An exemplary amino acid sequence of hPop1 is the sequence of human hPop1 as provided below:





















 (SEQ ID NO: 3)

It must be noted that, as used in this specification and the appended claims, the singular forms "a", "an" and "the" include plural referents unless the content clearly dictates otherwise. Thus, for example, reference to "a purified peptide" includes a mixture of two or more purified peptides, and the like.

The term "about," particularly in reference to a given quantity, is meant to encompass deviations of plus or minus five percent.

As used herein, the terms "includes," "including," "contains," "containing," and any variations thereof, are intended to cover a non-exclusive inclusion, such that a process, method, product-by-process, or composition of matter that includes, includes, or contains an element or list of elements does not include only those elements but can include other elements not expressly listed or inherent to such process, method, product-by-process, or composition of matter.

As used herein, the term "plurality" refers to a population of two or more members, such as peptide members or other referenced molecules. The two or more members of a plurality of members can be the same members or different members. For example, a plurality of peptides can include two or more peptide members having the same amino acid sequence. For another example, a plurality of peptides can include two or more peptide members having different amino acid sequences. A plurality can include 2, 3, 4, 5, 6, 7, 8, 9, 10, 15, 20, 25, 30, 35, 40, 45, or 50 different members. A plurality can also include 60, 70, 80, 90, 100 or more different members. A plurality includes all integer numbers in between the above exemplary plurality numbers.

The term "autoantibody" refers to an antibody or antibodies produced by one's immune system that targets the body's own constituents of the individual that produces the autoantibody. Normally, the immune system is able to recognize and ignore the body's own healthy proteins, cells, and tissues. Sometimes, however, the immune system ceases to recognize one or more of the body's normal constituents as "self," leading to production of pathological autoantibodies. These autoantibodies attack the body's own healthy cells, tissues, and/or organs, causing inflammation and damage. It should be noted that autoantibodies can also play a nonpathological role. For instance they can help the body to destroy cancers and to eliminate waste products.

The term "peptide," as used herein, includes an oligopeptide having between 2 and 30 amino acids *(e.g.,* 2, 3, 4, 5, 6, 7, 8, 9, 10, 12, 14, 16, 18, 20, 25 or 30 amino acids) as well as longer amino acid chains, for example, more than 30 amino acids, more than 50 amino acids, more than 100 amino acids, more than 150 amino acids, more than 200 amino acids, more than 300 amino acids, more than 400 amino acids, more than 500 amino acids, or more than 600 amino acids. The term "peptide" is used interchangeably with "polypeptide." A peptide can be produced, for example, recombinant expression, or by chemical synthesis. The peptide of this disclosure can be posttranslationally or chemically modified (e.g., carbamylation, phosphorylation, biotinylation, attachment of fluorescent dyes, and the like). A peptide can include unnatural amino acids that are not encoded by the natural genetic code. For example, a peptide can include methylated backbone structures, peptoid backbone structures (poly-N-substituted glycines), L-amino acids, R-amino acids, and the like. A peptide can have a wild-type sequence, naturally occurring variant sequence, mutant sequences (e.g., point mutants, deletion mutants), and the like. A peptide does not include a full length native protein.

The term "purified" as used herein, and grammatical equivalents thereof, unless specified otherwise, refer to the reduction in the amount of at least one contaminant (such as protein and/or nucleic acid sequence) from a sample or from a source from which the material is isolated. An "purified" peptide is substantially free of cellular material or other contaminating proteins from the cell or tissue source and/or other contaminant components from which the peptide is derived, or substantially free of chemical precursors or other chemicals when chemically synthesized. The language "substantially free of cellular material" includes preparations of an peptide in which the peptide is separated from cellular components of the cells from which it is isolated or recombinantly produced. Thus, a peptide that is substantially free of cellular material includes preparations of peptide having less than about 30%, 25%, 20%, 15%,10%, 5%, or 1% (by dry weight) of heterologous peptide (also referred to herein as a "contaminating peptide"). When the peptide is recombinantly produced, it is substantially free of culture medium, (e.g., culture medium represents less than about 20%, 15%, 10%, 5%, or 1% of the volume of the protein preparation). In certain embodiments, when the peptide is produced by chemical synthesis, it is substantially free of chemical precursors or other chemicals, for example, it is separated from chemical precursors or other chemicals which are involved in the synthesis of the protein. Accordingly such preparations of the peptide have less than about 30%, 25%, 20%, 15%, 10%, 5%, or 1% (by dry weight) of chemical precursors or compounds other than the peptide of interest.

The term "variant" when used in relation to a peptide or protein refers to a peptide or protein having one or more including, for example, two, three, four, five, six, seven, eight, nine, ten, eleven, twelve, fifteen, twenty, twenty five, thirty, thirty five, or forty amino acid sequence substitutions, deletions, and/or additions as compared to the reference sequence. Variants can be naturally occurring, such as allelic or splice variants, or can be artificially constructed. Peptide variants can be prepared by chemical synthesis or recombinantly from the corresponding nucleic acid molecules encoding the variants. A peptide variant can be encoded by a single nucleotide polymorphism (SNP) variant of a nucleic acid molecule that encodes Rpp25, Rpp38, or hPop1.

The term "native" when used in connection with biological materials such as nucleic acid molecules, proteins, host cells, and the like, refers to those which are found in nature and not manipulated, modified, and/or changed (*e.g.*, isolated, purified, selected) by a human being.

An amino acid residue/position can be modified when a change of a starting amino acid sequence takes place, wherein the change results from a sequence alteration involving the amino acid residue/positions. For example, amino acid modification include substitution of a residue with another amino acid (*e.g.,* a conservative or non-conservative substitution), insertion of one or more (*e.g*., fewer than 5, 4 or 3) amino acids adjacent to the residue/position, and deletion of the residue/position.

The term "anti-Th/To antibody" or "an antibody that binds to the Th/To complex" refers to an antibody that binds specifically to the Th/To complex, including at least one subunit of the Th/To complex, such as Rpp25, Rpp38 or hPop1. An anti-Th/To antibody can bind to an epitope of the Th/To complex. The anti-Th/To antibody can be an anti-Rpp25 antibody, an anti-Rpp38 antibody, an anti-hPop1 antibody, or a combination thereof. An "anti-Rpp25 antibody" or "an antibody that binds to Rpp25" refers to an antibody that binds specifically to a Rpp25 peptide, fragment, or epitope. An "anti-Rpp38 antibody" or "an antibody that binds to Rpp38" refers to an antibody that binds specifically to a Rpp38 peptide, fragment, or epitope. An "anti-hPop1 antibody" or "an antibody that binds to hPop1" refers to an antibody that binds specifically to a hPop1 peptide, fragment, or epitope. The extent of binding of an anti-Th/To antibody to an unrelated, non-Th/To protein can be less than about 10% of the binding of the antibody to the Th/To complex as measured, for example, by fluorescence activated cell sorting (FACS) analysis or an immunoassay such as a radioimmunoassay (RIA). An antibody that binds to the Th/To complex, as described herein can have a dissociation constant (Kd) of less than or equal to 10 nM, 9 nM, 8 nM, 7 nM, 6 nM, 5 nM, 4 nM, 0.9 nM, 0.8 nM, 0.7 nM, 0.6 nM, 0.5 nM, 0.4 nM, 0.3 nM, 0.2 nM, or 0.1 nM, and/or is greater than or equal to 0.1nM. The terms "binds" or "binding" refer to an interaction between molecules including, for example, to form a complex. Interactions can be, for example, non-covalent interactions including hydrogen bonds, ionic bonds, hydrophobic interactions, and/or van der Waals interactions. A complex can also include the binding of two or more molecules held together by covalent or non-covalent bonds, interactions or forces. The strength of the total non-covalent interactions between a single antigen-binding site on an antibody and a single epitope of a target molecule, such as Rpp25, Rpp38, or hPop1, is the affinity of the antibody or functional fragment for that epitope. The ratio of association (k1) to dissociation (k-1) of an antibody to a monovalent antigen (k1/ k-1) is the association constant K, which is a measure of affinity. The value of K varies for different complexes of antibody and antigen and depends on both k1 and k-1. The association constant K for an antibody provided herein can be determined using any method provided herein or any other method well known to those skilled in the art. The affinity at one binding site does not always reflect the true strength of the interaction between an antibody and an antigen.

With regard to the binding of an antibody to a target peptide, or the binding of a peptide to antibodies in a serum sample, the term "specific binding" or "specifically binds to" or is "specific for" means binding that is measurably different from a non-specific interaction. Specific binding can be measured, for example, by determining binding of a molecule compared to binding of a control molecule, which can be a molecule of similar structure that does not have binding activity. The term "specific binding" or "specifically binds to" or is "specific for" a particular peptide or an epitope on a particular peptide target as used herein can be exhibited, for example, by a molecule having a Kd for the target of at least about 10⁻⁴ M, alternatively at least about 10⁻⁵ M, alternatively at least about 10⁻⁶ M, alternatively at least about 10⁻⁷ M, alternatively at least about 10⁻⁸ M, alternatively at least about 10⁻⁹ M, alternatively at least about 10⁻¹⁰ M, alternatively at least about 10⁻¹¹ M, alternatively at least about 10⁻¹² M, or greater.

The specific binding between an antibody and an antigen, a peptide, or an epitope can be identified, for example, by immunoassays, Biacore, or other techniques known to those of skill in the art. An antibody binds specifically to a Th/To epitope when it binds to a Th/To epitope with higher affinity than to any cross reactive antigen as determined using experimental techniques, such as radioimmunoassays (RIA) and enzyme linked immunosorbent assays (ELISAs). A peptide that specifically binds to antibodies in the Th/To serum when it binds to the antibodies in the Th/To serum with higher affinity than to antibodies in a control serum. A specific or selective reaction can be at least twice background signal or noise or more than five or ten times background. *See* Paul, ed., 1989, Fundamental Immunology Second Edition, Raven Press, New York at pages 332-336 for a discussion regarding antibody specificity.

An "antigen" is a predetermined antigen to which an antibody can selectively bind. A target antigen can be a peptide, carbohydrate, nucleic acid, lipid, hapten or other naturally occurring or synthetic compound. The target antigen can be a peptide, including an autoantigen.

The term "epitope" as used herein refers to the site on the surface of a protein or an antigen molecule to which an antibody or functional fragment thereof binds. An epitope can be a localized region on the surface of an antigen such as a Rpp25 peptide, a Rpp38 peptide, or a hPop1 peptide, that can specifically bind to one or more antigen binding regions of an antibody or the functional fragment thereof. An epitope can be a separate molecule independent from the antigen or the protein that it is derived from. An epitope can be a peptide. An epitope is "derived from" a particular protein or antigen when it has the amino acid sequence of the site of the protein or antigen to which an antibody or functional fragment thereof binds, which is part of the sequence of the full length protein or antigen. For example, an epitope derived from Rpp25 contains the amino acid sequence of the site on the surface of Rpp25 to which an antibody or functional fragment thereof binds, and the amino acid sequence of the epitope is part of the amino acid sequence of Rpp25.

An epitope can be capable of eliciting an immune response. An epitope can have antigenic or immunogenic activity in an animal, such as a mammal (*e.g*., a human). An epitope having immunogenic activity is a portion of a peptide that elicits an antibody response in an animal. An epitope can consist of chemically active surface groupings of molecules such as amino acids or sugar side chains and can have specific three dimensional structural characteristics as well as specific charge characteristics. An epitope includes a linear epitope and a conformational epitope. A region of a peptide contributing to an epitope can be contiguous amino acids of the peptide or the epitope can come together from two or more non-contiguous regions of the peptide. The epitope can be a three-dimensional surface feature of the antigen. An antigen can have more than one different epitopes and can react with many different antibodies.

Epitope mapping is a process of identifying the binding sites, or epitopes, of antibodies on their target antigens. Antibody epitopes can be linear epitopes or conformational epitopes. Linear epitopes are formed by a continuous sequence of amino acids in a protein. Conformational epitopes are formed of amino acids that are discontinuous in the protein sequence, but which are brought together upon folding of the protein into its three-dimensional structure. Epitope mapping can be performed using solid phase peptides and other methods well known to those skilled in the art.

A peptide "reacts positively" with a sample when the sample contains antibodies that bind specifically to the peptide. The sample can be a serum sample. The serum can be obtained from a human subject. The term "a peptide that reacts positively with Th/To serum" refers to the peptide that specifically binds to antibodies in the Th/To serum. The term "Th/To serum" refers to the serum that contains anti-Th/To antibodies. The Th/To serum can be a pool of sera from at least two, three, four, five, or six individual samples.

A subject or a patient is "positive" for an autoantibody when the autoantibody can be detected above the noise of the respective assay from the sample of the subject or the patient. The sample of a subject or a patient can be a sample of blood, serum, or other bodily fluid including plasma, urine, or synovial fluid. The autoantibody can be anti-topoisomerase I antibodies (topo-I, Scl-70)(Mahler M et al, Autoimmun Rev 9:756-60(2010)), anti-centromere (CENP) antibodies (Fritzler MJ et al, Autoimmun Rev 10:194-200(2011)) and anti-RNA polymerase III (RNAP) antibodies (Mahler M et al, Ann N Y Acad Sci 1183:267-87 (2010); Van den Hoogen F et al, Arthritis Rheum 65:2737-47(2013)). The autoantibody can also be anti-PM/Scl complex (also known as the exosome) antibodies (Mahler M et al, Autoimmun Rev 6:432-7(2007)), anti-U3RNP/fibrillarin antibodies (Steen VD, Semin Arthritis Rheum 35:35-42(2005); Arnett FC et al, Arthritis Rheum 39:1151-60(1996)), and anti-Th/To complex antibodies. A subject is positive for an anti-Th/To complex antibody when the subject is positive for an antibody against at least one subunit of the Th/To complex. For example, a subject who is positive for an anti-Th/To complex antibody can be positive for an anti-Rpp25 antibody, an anti- Rpp38 antibody, an anti-hPopI antibody, or any combination thereof. A subject or a patient is "negative" for an autoantibody when the subject or patient is not "positive" for the autoantibody.

The autoantibodies can be detected by immunoprecipitation (IP) (Ceribelli A et al, J Rheumatol 37:2071-5(2010)), nucleolar immunofluorescence (IIF) staining, commercial line immunoassays (LI A) (Villalta D et al, Autoimmun Rev 12: 114-20 (2012); Bonroy C et al, J Immunol Methods 379:53-60(2012)), IP real-time PCR assay (Ceribelli A et al, Arthritis Res Ther 14:R128(2012)), or chemiluminescent immunoassay.

The "antinuclear antibody (ANA) test" is used as a primary test to help evaluate a person for systemic autoimmune disorders. Different test methods are known for the detection of ANA. Two common methods include solid phase immunoassay (SPA) and the indirect fluorescent assay (IF A) on HEp-2 cells (Mahler M et al, Current Concepts and Future Directions for the Assessment of Autoantibodies to Cellular Antigens Referred to as Anti-Nuclear Antibodies, J Immunol Res. 2014:315179 (2014)). A subject or a patient has a "positive ANA result" means that ANA is detected by at least one of the methods in the subject or patient's sample, including a blood sample. A subject or a patient has a "negative ANA result" when at least one of the methods fails to detect ANA in the sample from the subject or patient.

The terms "subject" can be an individual, who can be a patient. The subjects of this disclosure include healthy subjects, asymptomatic subjects, and diseased subjects. Diseased subjects can suffer from any disease associated with an aberrant anti-Th/To autoantibody level. The term "aberrant anti-Th/To autoantibody level", as used herein, refers to anti-Th/To autoantibody level in a subject that significantly deviate from the median anti-Th/To autoantibody level found in a population of healthy subjects. In some embodiments, the aberrant anti-Th/To antibody level is higher than the median anti-Th/To autoantibody level.

Herein, epitopes of the Th/To complex are identified based on peptides of Rpp25, Rpp38, or hPopl that react positively with serum containing anti-Th/To antibodies, and negatively with disease control serum lacking anti-Th/To antibodies ("positive peptides"; SEQ ID NOs: 4-80 in Table 2). Described herein is a purified peptide that contains any one of the peptides exemplified in Table 2. In one aspect,
described herein is a purified peptide having the amino acid sequence of SEQ ID NO: 4; in another aspect, described herein is a purified peptide having the amino acid sequence of SEQ ID NO: 33.

Table 3 lists the novel epitopes of the Th/To complex identified herein (SEQ ID NOS: 14, 15, 21, 35, 36, 49, 53, 60-62. 65, 66, 71, 72, and 77-97). The epitopes are identified based on information of the positive peptides as listed in Table 2. For example, epitope No. 2 of Rpp25 (SEQ ID NO: 82) is identified based on the positive peptides of SEQ ID NOS 6-13. For another example, epitope No. 4 of Rpp38 (SEQ ID NO: 35) is identified based on the positive peptide of the SEQ ID NO 35. Some epitopes (SEQ ID NOS: 14, 15, 21, and 81- 84) are derived from Rpp25; some epitopes (SEQ ID NOS: 35, 36, 49, 53, and 85-92) are derived from Rpp38; and the rest epitopes (SEQ ID NOS: 60-62, 65, 66, 71, 72, 77-80, and 93- 97) are derived from hPopl. Described herein is a purified peptide having at least seven contiguous amino acids of any one of the identified epitopes or a variant thereof as listed in Table 3. In one aspect, for example, the purified peptide can include the amino acid sequence of ATASMAQ (SEQ ID NO: 105), which are seven contiguous amino acids of epitope region No. 1 of Rpp25 (see Table 3). In another aspect, the purified peptide can include the amino acid sequence of QVSGWTPAHVRKQL (SEQ ID NO: 106), which are 14 contiguous amino acid of epitope region No. 7 of Rpp38 (see Table 3). These and other peptides as described below can be used to detect autoantibodies against the Th/To complex and to diagnose diseases related to the anti-Th/To complex autoantibodies.

Described herein is a purified peptide having at least seven contiguous amino acids of an epitope derived from one subunit of the Th/To complex, wherein the purified peptide reacts positively with human serum having anti-Th/To antibodies, but negatively with human sera lacking anti-Th/To antibodies; wherein the subunit of the Th/To complex is Rpp25, Rpp38, or hPopl . An epitope derived from a subunit of the Th/To complex has an amino acid sequence that is part of the amino acid sequence of subunit.

A purified peptide can be a purified recombinant peptide encoded by cDNA or a chemically synthesized peptide. Methods for expressing and purifying recombinant peptides are well known in the art. For example, a recombinant peptide can be expressed in and purified from bacterial cells (e.g., E.co/z), yeast cells (e.g., S. cerevisiae), in mammalian cells (e.g., CHO) and others. A recombinant peptide can be expressed and purified as fusion proteins including
tags for protein detection or affinity purification tags (e.g., His-tag, GST-tag, Myc-tag), including cleavable tags (e.g., tags including a TEV-cleavage site).

Exemplary methods for expressing and purifying a recombinant peptide, and for chemically synthesizing a peptide can be found, for example, in Scopes R.K., Protein Purification - Principles and Practice, Springer Advanced Texts in Chemistry, 3rd Edition (1994); Simpson R.J. et al., Basic Methods in Protein Purification and Analysis: A Laboratory Manual, Cold Spring Harbor Laboratory Press, 1st Edition (2008); Green M.R. and Sambrook J., Molecular Cloning: A Laboratory Manual, Cold Spring Harbor Laboratory Press, 4st Edition (2012); Jensen K.J. et al, Peptide Synthesis and Applications (Methods in Molecular Biology), Humana Press, 2nd Edition (2013).

In some aspects, the purified peptide includes unnatural amino acids. In some aspects, the unnatural amino acids are methylated at the a-amino-group to produce peptides with methylated backbones. In some embodiments, the unnatural amino acids are R-amino acids. In some aspects, the unnatural amino acid can include a dye (e.g., a fluorescent dye) or an affinity tag. In some aspects, the purified peptide includes chemical modification. Chemical modifications include, for example, chemical modifications with biotin, fluorescent dyes. A skilled artisan will recognize that methods for introducing unnatural amino acids into a peptide and for chemically modifying a peptides are well known in the art.

Described herein is a purified peptide having at least seven contiguous amino acids of an epitope derived from one subunit of the Th/To complex, wherein the epitope has an amino acid sequence selected from the group consisting of SEQ ID NOS: 14, 15, 21, 35, 36, 49, 53, 60-62. 65, 66, 71, 72, and 77-97, or a variant thereof. In some aspects, the purified peptide has an amino acid sequence selected from the group consisting of SEQ ID NOS: 4-80

Also described herein is an epitope variant that contains an epitope for a Th/To antibody and has at least seven contiguous amino acids having at least 85%, 90%>, 95%, 96%), 97%), 98%o, or 99% sequence identity with an amino acid sequence selected from the group consisting of SEQ ID NOS: 14, 15, 21, 35, 36, 49, 53, 60-62 65, 66, 71, 72, and 77-97.

In some aspects, the at least seven contiguous amino acids are at least eight, night, ten, eleven, twelve, thirteen, fourteen, fifteen, sixteen, seventeen, eighteen, nineteen, or twenty amino acids. In some aspects, the at least seven contiguous amino acids are seven, eight, night, ten, eleven, twelve, thirteen, fourteen, fifteen, sixteen, seventeen, eighteen, nineteen, or twenty amino acids. In some aspects, the at least seven contiguous amino acids can be fifteen amino acids.

In some aspects, the purified peptide described herein has at least seven, eight, nine, ten, eleven, twelve, thirteen, fourteen, fifteen, sixteen, seventeen, eighteen, nineteen, or twenty amino acids. In one aspect, the purified peptide described herein has fifteen amino acids.
In some aspects, the purified peptide has at most 30, 40, 50, 60, 70, 80, 90, 100, 125, 150, 175, 200, 300, 400, 500 amino acids.

In some other aspects, the purified peptide has between seven and 500 amino acids, between seven and 400 amino acids, between seven and 300 amino acids, between seven and 200 amino acids, between seven and 100 amino acids, between seven and 50 amino acids, between ten and 50 amino acids, between ten and 40 amino acids, or between ten and 30 amino acids.

The novel epitopes identified herein are derived from a subunit of the Th/To complex, including Rpp25, Rpp38, and hPopl . Epitopes of SEQ ID NOS: 14, 15, 21, and 81-84 are derived from Rpp25; epitopes of SEQ ID NOS: 35, 36, 49, 53, and 85-92 are derived from Rpp38; epitopes of SEQ ID NOS: 60-62, 65, 66, 71, 72, 77-80, and 93-97 are derived from hPopl . The amino acid sequence of an epitope derived from Rpp25 (or Rpp38, or hPopl) is part of the sequence of Rpp25 (or Rpp38, or hPopI).

In some aspects, described herein is a purified peptide having at least seven contiguous amino acids of an epitope derived from a subunit of the Th/To complex that includes Rpp25, wherein the epitope has an amino acid sequence selected from the group consisting of SEQ ID NOS: 14, 15, 21, and 81-84. In some aspects, described herein is a purified peptide having at least seven contiguous amino acids of an epitope derived from a subunit of the Th/To complex that includes Rpp38, wherein the epitope has an amino acid sequence selected from the group consisting of SEQ ID NOS: 35, 36, 49, 53, and 85-92. In other aspects, described herein is a purified peptide having at least seven contiguous amino acids of an epitope derived from a subunit of the Th/To complex that includes hPopl, wherein the epitope has an amino acid sequence selected from the group consisting of SEQ ID NOS: 60- 62, 65, 66, 71, 72, 77-80, and 93-97.

Also described herein is a composition of more than one purified peptides that are derived from epitopes of the Th/To complex. In some aspects, described herein is a purified peptide having at least seven contiguous amino acids of an epitope derived from one subunit of the Th/To complex, wherein the epitope has an amino acid sequence selected from the group consisting of SEQ ID NOS: 14, 15, 21, 35, 36, 49, 53, 60-62. 65, 66, 71, 72, and 77-97; and wherein the purified peptide is a plurality of purified peptides.

In some aspects, the plurality of purified peptides each contains at least seven contiguous amino acids of an epitope derived from the same subunit of the Th/To complex, which can be Rpp25, Rpp38 or hPopl. In some aspects, described herein is a plurality of purified peptides having a first purified peptide and a second purified peptide, wherein the first purified peptide and the second purified peptide each has at least seven contiguous amino acids of an epitope derived from Rpp25, the epitope having an amino acid sequence selected from the group consisting of SEQ ID NOS: 14, 15, 21, and 81-84. For example, the plurality of purified peptides can include two different purified peptides that have the sequences of AFATASMAQPATRAIVFSGC (SEQ ID NO: 99) and EPGVADEDQT (SEQ ID NO: 107), which are derived from epitope regions No. 1 and No. 3 of Rpp25, respectively (see Table 3). For another example, a plurality of purified peptides can have two different purified peptides that have the sequences of TQGQTPGEPAAS (SEQ ID NO: 108) and LAILLSKDALDPRQPG (SEQ ID NO: 109), both derived from epitope region No. 2 of Rpp25. (see Table 3). Other exemplary pluralities of peptides are described further below.

In some aspects, described herein is a plurality of purified peptides having a first purified peptide and a second purified peptide, wherein the first purified peptide and the second purified peptide each has at least seven contiguous amino acids of an epitope derived from Rpp38, the epitope having an amino acid sequence selected from the group consisting of SEQ ID NOS: 35, 36, 49, 53, and 85-92.

In some other aspects, described herein is a plurality of purified peptides having a first purified peptide and a second purified peptide, wherein the first purified peptide and the second purified peptide each has at least seven contiguous amino acids of an epitope derived from hPopI, the epitope having an amino acid sequence selected from the group consisting of SEQ ID NOS: 60-62, 65, 66, 71, 72, 77-80, and 93-97.

In some aspects, the purified peptide described herein is a plurality of purified peptides derived from different protein subunits. In some aspects, the plurality of purified peptides have a first purified peptide and a second purified peptide, wherein the first purified peptide has at least seven contiguous amino acids of a first epitope derived from Rpp25, the first epitope having an amino acid sequence selected from the group consisting of SEQ ID NOS: 14, 15, 21, and 81-84; and the second purified peptide has at least seven contiguous amino acids of a second epitope derived from Rpp38, the second epitope having an amino acid sequence selected
from the group consisting of SEQ ID NOS: 35, 36, 49, 53, and 85-92. For example, the plurality of purified peptides can include two different purified peptides that have the sequences of EPGVADEDQTA (SEQ ID NO: 110) and RELLDTSFEDLSKPK(SEQ ID NO: 25), which are derived from epitope regions No. 2 of Rpp25, and epitope region No. 1 of Rpp38, respectively (see Table 3).

In some aspects, the plurality of purified peptides have a first purified peptide and a second purified peptide, wherein the first purified peptide has at least seven contiguous amino acids of a first epitope derived from Rpp25, the first epitope having an amino acid sequence selected from the group consisting of SEQ ID NOS: 14, 15, 21, and 81-84; and the second purified peptide has at least seven contiguous amino acids of a second epitope derived from hPopl, the second epitope having an amino acid sequence selected from the group consisting of SEQ ID NOS: 60-62, 65, 66, 71, 72, 77-80, and 93-97.

In some other aspects, the plurality of purified peptides have a first purified peptide and a second purified peptide, wherein the first purified peptide has at least seven contiguous amino acids of a first epitope derived from hPopI, the first epitope having an amino acid sequence selected from the group consisting of SEQ ID NOS: 60-62, 65, 66, 71, 72, 77-80, and 93-97; and the second purified peptide has at least seven contiguous amino acids of a second epitope derived from Rpp38, the second epitope having an amino acid sequence selected from the group consisting of SEQ ID NOS: 35, 36, 49, 53, and 85-92.

In some other aspects, the plurality of purified peptides have a first purified peptide, a second purified peptide, and a third purified peptide, wherein the first purified peptide has at least seven contiguous amino acids of a first epitope derived from Rpp25, the first epitope having an amino acid sequence selected from the group consisting of SEQ ID NOS: 14, 15, 21, and 81- 84; the second peptide has at least seven contiguous amino acids of a second epitope derived from Rpp38, the second epitope having an amino acid sequence selected from the group consisting of SEQ ID NOS: 35, 36, 49, 53, and 85-92; and the third purified peptide has at least seven contiguous amino acids of a third epitope derived from hPopI, the third epitope having an amino acid sequence selected from the group consisting of SEQ ID NOS: 60-62, 65, 66, 71, 72, 77-80, and 93-97.

As such, described herein is a plurality of at least two, three, four, five, six, or seven purified peptides, each having at least seven contiguous amino acids of one of the
identified epitopes exemplified in Table 3. The disclosure includes any and all types of combinations of the purified peptides, each having at least seven contiguous amino acids of one of the identified epitopes exemplified in Table 3. In some embodiments, the at least seven contiguous amino acids are at least eight, night, ten, eleven, twelve, thirteen, fourteen, fifteen, sixteen, seventeen, eighteen, nineteen, or twenty amino acids. In other embodiments, the disclosure also includes any and all types of combinations of the purified peptides, each having an amino acid sequence selected from those exemplified in Table 2, including SEQ ID NO: 4-97. The plurality of purified peptides can be more efficient in binding to and detecting the autoantibodies against the Th/To complex as compared to a single purified peptide with one epitope region.

Also described herein is a purified peptide containing at least two regions in a single molecule, wherein each epitope region contains at least seven contiguous amino acid sequence of one of the epitopes of the Th/To complex identified in this application. In some aspects, described herein is a purified peptide including a first region of at least seven contiguous amino acids of a first epitope having an amino acid sequence selected from the group consisting of SEQ ID NOS: 14, 15, 21, 35, 36, 49, 53, 60-62. 65, 66, 71, 72, and 77-97, and a second region of at least seven contiguous amino acids of a second epitope having an amino acid sequence selected from the group consisting of SEQ ID NOS: 14, 15, 21, 35, 36, 49, 53, 60-62. 65, 66, 71, 72, and 77-97.

The at least two regions of a single purified peptide can contain the same amino acid sequences or different amino acid sequences. When the two regions contain different amino acid sequence, the first epitope and the second epitope can be derived from either the same subunit or different subunits of the Th/To complex. The amino acid sequence of an epitope derived from a particular subunit of the Th/To complex is part of the amino acid sequence of the subunit.

In some aspects, the first epitope and the second epitope are derived from the same subunit of the Th/To complex selected from the group consisting of Rpp25, Rpp38 and hPopl . In one aspect, the first epitope and the second epitope are derived from Rpp25; and described herein is a purified peptide having a first region of at least seven contiguous amino acids of a first epitope and a second region of at least seven contiguous amino acids of a second epitope, both the first epitope and the second epitope having an amino acid sequence selected from the group consisting of SEQ ID NOS: 14, 15, 21, and 81-84. In another aspect, the
first epitope and the second epitope are derived from Rpp38; and described herein is a purified peptide having a first region of at least seven contiguous amino acids of a first epitope and a second region of at least seven contiguous amino acids of a second epitope, both the first epitope and the second epitope having an amino acid sequence selected from the group consisting of SEQ ID NOS: 35, 36, 49, 53, and 85-92. In yet another aspect, the first epitope and the second epitope are derived from hPopl; and described herein is a purified peptide having a first region of at least seven contiguous amino acids of a first epitope and a second region of at least seven contiguous amino acids of a second epitope, both the first epitope and the second epitope having an amino acid sequence selected from the group consisting of SEQ ID NOS: 60-62, 65, 66, 71, 72, 77-80, and 93-97. For example, the purified peptide can have the sequence of:
(x)ₘ-ATASMAQPATRA-(x)ₙ-KNVPGLAILLSKDALD-(x)ₗ (SEQ ID NO: 111) which includes 12 contiguous amino acids of the epitope region No. 1 of Rpp25 (SEQ ID NO: 81) and 16 contiguous amino acids of the epitope region No. 2 of Rpp25 (SEQ ID NO: 82). Here, x can be any amino acid; m, n and I represents the number of the random amino acids "x" and can be any integral between 0-50, 0-100, or 0-150.

For another example, the purified peptide can have the sequence of TASMAQPATRAIVFS-AASLSVLKNVPGLAI (SEQ ID NO: 100), which includes 15 contiguous amino acids of the epitope region No. 1 of Rpp25 (SEQ ID NO. 81) and 15 contiguous amino acids of the epitope region No. 15 of Rpp25 (SEQ ID NO. 15).

In some other aspects, the first epitope and the second epitope are derived from two different subunits of the Th/To complex selected from the group consisting of Rpp25, Rpp38 and hPopl . In one aspect, the first epitope region is derived from Rpp25 and the second epitope region is derived from Rpp38. For example, the purified peptide can have the sequence of:
(x)ₘ-ATASMAQPATRA-(x)ₙ-PYIIRWSALESEOM-(x)ₗ (SEQ ID NO: 112), which includes 12 contiguous amino acids of the epitope region No. 1 of Rpp25 (SEQ ID NO: 81) and 14 contiguous amino acids of the epitope region No. 2 of Rpp38 (SEQ ID NO: 89). Here, x can be any amino acid; m, n and I represents the number of the random amino acids "x" and can be any integral between 0-50, 0-100, or 0-150.

For another example, the purified peptide can have the sequence of DTSFEDLSKPKRKLA-TGIIISDLTMEMNRF (SEQ ID NO: 104), which includes 15 contiguous amino acids of the epitope region No. 11 of Rpp38 (SEQ ID NO: 91) and 15 contiguous amino acids of the epitope region No. 7 of hPop1 (SEQ ID NO: 94).

In another aspect, first epitope region is derived from Rpp25 and the second epitope region is derived from hPopl . In yet another aspect, the first epitope region is derived from hPopl and the second epitope region is derived from Rpp38.

As such, described herein is a purified peptide with at least two, three, four, or five regions for binding to autoantibodies against the Th/To complex, each region having at least seven contiguous amino acids of one of the epitopes identified in Table 3. The disclosure includes any and all types of combinations of the regions, each having at least seven contiguous amino acids of one of the identified epitopes exemplified in Table 3. In some aspects, the at least seven contiguous amino acids are at least eight, night, ten, eleven, twelve, thirteen, fourteen, fifteen, sixteen, seventeen, eighteen, nineteen, or twenty amino acids. The disclosure also includes any and all types of combinations of the regions, each having an amino acid sequence selected from the sequences of the positive peptides exemplified in Table 2. The purified peptide with two or more epitope regions can be more efficient in binding to and detecting the autoantibodies against the Th/To complex as compared to a single purified peptide with one epitope region.

Different epitopes vary in their efficiencies in detecting the autoantibodies against the Th/To complex. Also described herein are eight peptides designed based on the identified epitopes with high efficiency in detecting the autoantibodies against the Th/To complex (see Example I below). The designed peptides are listed in Table 4 below (SEQ ID NOS: 25, 98-104). In some aspects, described herein is a purified peptide having at least seven contiguous amino acids having at least 80%, 85%, 90%>, 95%, 96%, 97%, 98%), or 99%) sequence identity with an amino acid sequence selected from the group consisting of SEQ ID NOS: 25, 98-104. In some aspects, described herein is a purified peptide having at least seven contiguous amino acids having the sequence selected from the group consisting of SEQ ID NOS: 25, 98-104. In some aspects, described herein is a purified peptide having the amino acid sequence selected from the group consisting of SEQ ID NO: 25, 98-104. In one aspect, described herein is a purified peptide having the amino acid sequence of SEQ ID NO: 25. In another aspect, described herein is a purified peptide having the amino acid sequence of SEQ ID NO: 102. In yet another aspect,
described herein is the purified peptide of the amino acid sequence of SEQ ID NO: 25 or SEQ ID NO: 102.

The purified peptide of this disclosure can specifically bind to an anti-Th/To antibody. When the binding happens, a complex is formed between the purified peptide and the anti-Th/To antibody. The anti-Th/To antibody is an anti-Rpp25 antibody, an anti-Rpp38 antibody, an anti-hPopl antibody, or any combination thereof.

In some aspects, also described herein is a complex including a purified peptide of this disclosure and one or more anti-Th/To antibodies. In some aspects, the purified peptide has at least seven contiguous amino acids of an epitope having an amino acid sequence selected from the group consisting of SEQ ID NOS: 14, 15, 21, 35, 36, 49, 53, 60-62. 65, 66, 71, 72, and 77-97 or a variant thereof. The at least seven contiguous amino acids can be at least eight, night, ten, eleven, twelve, thirteen, fourteen, fifteen, sixteen, seventeen, eighteen, nineteen, or twenty amino acids. In other aspects, the purified peptide has an amino acid sequence selected from the group consisting of SEQ ID NOS: 4-80, and 98-104. In one aspect, the purified peptide of the complex has the amino acid sequence of SEQ ID NO: 25. In another aspect, the purified peptide of the complex has the amino acid sequence of SEQ ID NO: 102. The complex can be in solution or immobilized on a surface.

The purified peptide of the disclosure can be used for detecting an anti-Th/To antibody. The method of detecting an anti-Th/To antibody can be applied in the diagnosis or prognosis of SSc and any other autoimmune disease related to the Th/To autoantibody. It can also be applied in monitoring the efficacy of treatment in a patient with SSc, or any other autoimmune disease related to the Th/To autoantibody.

In some aspects, described herein is a method for detecting an anti- Th/To antibody in a subject including: a) contacting a sample from the subject with a purified peptide having at least seven contiguous amino acids of an epitope derived from a subunit of a Th/To complex, wherein the epitope having an amino acid sequence selected from the group consisting of SEQ ID NOS: 14, 15, 21, 35, 36, 49, 53, 60-62. 65, 66, 71, 72, and 77-97, or a variant thereof, to form a complex between an anti-Th/To antibody and the purified peptide; and b) detecting the presence or absence of the anti- Th/To antibody-purified peptide complex in the sample; the anti-Th/To antibody is an anti-Rpp25 antibody, an anti-Rpp38 antibody, an anti- hPopl antibody, or any combination thereof.

In some aspects, the at least seven contiguous amino acids can be at least eight, night, ten, eleven, twelve, thirteen, fourteen, fifteen, sixteen, seventeen, eighteen, nineteen, or twenty amino acids. In some aspects, the purified peptide has an amino acid sequence selected from the group consisting of SEQ ID NOS: 4-80 and 98-104 . In some aspects, the purified peptide has the amino acid sequence of SEQ ID NO: 25. In other aspects, the purified peptide has the amino acid sequence of SEQ ID NO: 102.

In one aspect, the anti-Th/To antibody is an anti-Rpp25 antibody; in one aspect, the anti-Th/To antibody is an anti-Rpp38 antibody; in one aspect, the anti-Th/To antibody is an anti- hPopl antibody. In another aspect, the anti-Th/To antibody is an anti-Rpp25 antibody and an anti-Rpp38 antibody; in another aspect, the anti-Th/To antibody is an anti-Rpp25 antibody and an anti-hPopl antibody; in another aspect, the anti-Th/To antibody is an anti-hPopl antibody and an anti-Rpp38 antibody. In yet another aspect, the anti-Th/To antibody is an anti-Rpp25 antibody, an anti-Rpp38 antibody, and an anti-hPopI antibody.

In some embodiments, described herein is a method for detecting an anti- Th/To antibody, wherein the presence or absence of the anti-Th/To antibody-purified peptide complex is detected by an assay selected from the group consisting of an enzyme-linked immunosorbent assay (ELISA), a fluorescent immunosorbent assay (FIA), a chemiluminescent immunosorbent assay (CLIA), a radioimmunoassay (RIA), an enzyme multiplied immunoassay, a solid phase radioimmunoassay (SPROA), a fluorescence polarization (FP) assay, a fluorescence resonance energy transfer (FRET) assay, a time-resolved fluorescence resonance energy transfer (TR-FRET) assay and a surface plasmon resonance (SPR) assay.

In some embodiments, the ELISA is a sandwich ELISA. In some embodiments, the sandwich ELISA includes the initial step of immobilizing a purified peptide of this disclosure on a solid support (e.g., on the wall of a microtiter plate well or of a cuvette). In some embodiments, contacting the sample from the subject with the purified peptide of this disclosure includes exposing the sample to the immobilized purified peptide.

In some embodiments, the ELISA is a direct ELISA. In some embodiments, the direct ELISA includes the initial step of immobilizing the anti-Th/To antibody in the sample on a solid support (e.g., on the wall of a microtiter plate well or of a cuvette). In some embodiments, contacting the sample from the subject with the purified peptide of this disclosure includes exposing a purified peptide of this disclosure to the immobilized the anti-Th/To antibody.

In some embodiments, the presence or absence of the anti-Th/To antibody-peptide complex is detected concurrently with the presence or absence of another analyte (e.g., another biomarker or disease marker) in a multiplex assay.

Methods and protocols for conducting immunoassays and biophysical protein-interaction assays are well known in the art. *See e.g.,* Wild D., The Immunoassay Handbook, Elsevier Science, 4th Edition (2013); Fu H., Protein-Protein Interactions, Humana Press, 4th Edition (2004).

In some embodiments, the method for detecting an anti-Th/To antibody can be performed according to the following protocol. First, a purified peptide of this disclosure and a negative control peptide with random sequence is diluted in coating buffer to prepare 10µg/ml solutions. 50µl of the peptide solution is dispensed into positive control wells and test wells of a 96-well microtiter plate. 50µl of the peptide solution is dispensed into the negative control wells on the same 96-well microtiter plate. The microtiter plate and peptide solutions are incubated overnight at 4°C. Next, 100µl blocking buffer are added to the positive control, negative control and test wells of the microtiter plate and the plate is incubated for an additional 6 hours at 4°C. At the end of the incubation period, the plate is washed three times with washing buffer. Serum test samples (having unknown anti-Th/To antibody contents) are diluted 50-fold in dilution buffer; positive control standards are prepared using serum samples known to contain anti-Th/To antibodies (e.g., as single concentration standards or dilution series) and negative control samples are prepared using dilution buffer alone or serum samples known not to contain anti-Th/To antibodies. After removing the washing buffer from the microtiter plate, 50µl of the test samples, positive control samples, and negative control samples are added to the test , negative control and positive control wells on the microtiter plate, respectively. Next, the microtiter plate is incubated overnight at 4°C on ice. On the next day, the microtiter plate is washed three times with washing buffer. Rabbit anti-human-IgG-HRP is diluted 1:5,000 in dilution buffer and 50µl of the antibody-conjugate is added to each microtiter plate well after removing the washing buffer. After 3.5 hours incubation at 4°C on ice the microtiter plate is wash another three times with washing buffer. A detection substrate solution is prepared by adding 5µl H₂O₂ to per 10ml ABTS solution (concentration according to manufacturer's instructions). 50µl of the detection substrate solution is added to each microtiter plate well after removing the washing buffer. The microtiter plate is then incubated in the dark at room temperature for 0.5-5 min and read on an ELISA reader. The relative absorbance signals for the negative control wells (e.g., average or median signals) are subtracted from the signals obtained for the test well and positive control wells. Test serum samples resulting in significant absorbance signals above background (e.g., *2* standard deviations (STDs) above the negative control well signals) are considered anti-Th/To antibody positive. The level of anti-Th/To antibodies can be quantified in anti-Th/To antibody positive samples by comparing the relative absorbance signals of the test wells with the absorbance signals observed for the positive control cells.

In some embodiments, the methods of this disclosure are performed, at least in part, using one or more automated assay systems. In some embodiments, the automated assay system include, for example, a BIO-FLASH^{™}, a BEST 2000^{™}, a DS2^{™}, an ELx50 WASHER, an ELx800 WASHER, an ELx800 READER, and an Autoblot S20^{™} (INOVA Diagnostics, Inc., San Diego, CA).

In some embodiments, the method for detecting an anti-Th/To antibody in a subject further includes the initial step of preparing the purified peptide. The purified peptide can be prepared by chemical synthesis or recombinant production. Exemplary methods for expressing and purifying a recombinant peptide can be found, for example, in Scopes R.K., Protein Purification - Principles and Practice, Springer Advanced Texts in Chemistry, 3rd Edition (1994); Simpson R.J. et al., Basic Methods in Protein Purification and Analysis: A Laboratory Manual, Cold Spring Harbor Laboratory Press, 1st Edition (2008); Green M.R. and Sambrook J., Molecular Cloning: A Laboratory Manual, Cold Spring Harbor Laboratory Press, 4st Edition (2012); Jensen K.J. et al., Peptide Synthesis and Applications (Methods in Molecular Biology), Humana Press, 2nd Edition (2013). Chemically synthesis of a peptide can be accomplished by using methodologies well known in the art (see Kelley and Winkler, 1990, In: Genetic Engineering Principles and Methods, Setlow, J. K, ed., Plenum Press, N.Y., Vol. 12, pp 1-19; Stewart, et al., 1984, J. M. Young, J. D., Solid Phase Peptide Synthesis, Pierce Chemical Co., Rockford, Ill; A. Marglin and R.B. Merrifield, Chemical Synthesis of Peptides and Proteins Ann. Rev. Biochem, 39:841-866, at 862 (1970). Merrifield, R.B., 1963, J. Am. Chern. Soc. 85:2149-2154; Chemical Approaches to the Synthesis of Peptides and Proteins, Williams et al., Eds., 1997, CRC Press, Boca Raton Fla.; Solid Phase Peptide Synthesis: A Practical Approach, Atherton & Sheppard, Eds., 1989, IRL Press, Oxford, England; See also U.S. Pat. Nos. 4,105,603; 3,972,859; 3,842,067; and 3,862,925).

In some embodiments, the method further includes immobilizing the purified peptide on a solid surface, or a solid support. In some embodiments, the purified proteins are immobilized via a link molecule coupling the purified protein to the solid support. When referring to immobilization of molecules (e.g., purified proteins) to a solid support, the terms "immobilized" and "attached" are used interchangeably herein and both terms are intended to encompass direct or indirect, covalent or non-covalent attachment, unless indicated otherwise, either explicitly or by context. In some embodiments, covalent attachment is involved, but all that is required is that the molecules remain immobilized or attached to the support under the conditions in which it is intended to use the support, for example, in applications requiring antibody-binding or detection.

The solid surface or solid support can be any material that is appropriate for or can be modified to be appropriate for the attachment of the purified peptide of this disclosure. As will be appreciated by those in the art, the number of possible substrates is very large. Possible substrates include, but are not limited to, glass and modified or functionalized glass, plastics (including acrylics, polystyrene, polyurethanes, Teflon^{™}, etc.), polysaccharides, nylon or nitrocellulose, ceramics, resins, silica or silica-based materials including silicon and modified silicon, carbon metals, inorganic glasses, optical fiber bundles, and a variety of other polymers. In some embodiments, the solid supports are located in microtiter well plates *(e.g.,* a 96-well, 384-well or 1536-well plate). In some embodiments, the solid supports are located within a flow cell or flow cell apparatus (e.g., a flow cell on a Biacore^{™} chip or a protein chip).

In some embodiments, the solid support includes a patterned surface suitable for immobilization of purified peptide in an ordered pattern *(e.g.,* a peptide chip). A patterned surface refers to an arrangement of different regions in or on an exposed layer of a solid support. For example, one or more of the regions can be features where one or more purified peptides are present. The features can be separated by interstitial regions where purified peptids are not present. In some embodiments, the pattern can be an x-y format of features that are in rows and columns. In some embodiments, the pattern can be a repreating arrangement of features and/or interstitial regions. In some embodiments, the pattern can be a random arrangement of features and/or inteststitial regions. Exemplary patterned surfaces that can be used in the methods and compositions set forth herein are described in U.S. Pat. App. Publ. No. 2008/0280785 A1, U.S. Pat. App. Publ. No. 2004/0253640 A1, U.S. Pat. App. Publ. No. 2003/0153013 A1 and International Publication No. WO 2009/039170 A2.

In some embodiments, the solid support includes an array of wells or depressions in a surface. This can be fabricated using a variety of techniques known in the art, including, but not limited to photolithography, stamping techniques, molding techniques and microetching techniques. As will be appreciated by those skilled in the art, the technique used will depend on the composition and shape of the array substrate.

In some embodiments, the solid support or its surface is non-planar, such as the inner or outer surface of a tube or vessel. In some embodiments, the solid support includes microspheres or beads. By "microspheres" or "beads" or "particles" or grammatical equivalents herein is meant small discrete particles. Suitable bead compositions include, but are not limited to, plastics, ceramics, glass, polystyrene, methylstyrene, acrylic polymers, paramagnetic materials, thoria sol, carbon graphite, titanium oxide, latex or cross linked dextrans such as Sephadose, cellulose, nylon, cross-linked micelles and Teflon^{™}, as well as any other materials outlined herein for solid supports can all be used. "Bangs Beads Techincal Product Guide" from Bangs Laboratories (Fishers, Ind) is a helpful guide. In some embodiments, the microspheres are magnetic microspheres or beads.

The beads need not be spherical; irregular particles can be used. Alternatively or additionally, the beads can be porous. The bead sizes range from nanometers, for example, 100nm, to millimeters, for example, 1 mm. In some embodiments, the bead sizes range from about 0.2 to about 200 microns. In some embodiments, bead sizes range from about 0.5 to about 5 microns. In some embodiments beads smaller than about 0.2 microns or larger than about 200 microns can be used.

In some embodiments, the method for detecting an anti-Th/To antibody in a subject further includes obtaining the sample from the subject. In some embodiments, the sample is a plurality of samples. In some embodiments, the sample is a blood sample, a plasma sample, a serum sample, a synovial fluid sample or another tissue or bodily fluid sample. In some embodiments, a plurality of samples are obtained over a period of time, which can be more than 12 hours, more than 1 day, more than 2 days, more than 3 days, more than 4 days, more than 5 days, more than 6 days, more than 7 days, more than 10 days, more than 14 days, more than 3 weeks, more than 1 month, more than 2 months, more than 3 months, more than 4 months, more than 5 months, more than 6 months, more than 9 months, more than 12 months, more than 18 months, more than 24 months, more than 30 months, more than 3 years months, more than 4 years, or more than 5 years. In some aspects, the sample is obtained freshly from a subject or patient; in other aspects, the sample has been cryopreserved and thawed before being tested.

In some embodiments, one or more samples were obtained before the subject received an SSc treatment (e.g., a drug regimen to treat or prevent SSc). In some embodiments, one or more samples are obtained after the subject received an SSc treatment or during the course of an ongoing SSc treatment period.

A number of autoimmune diseases have been associated with the presence of autoantibody against the Th/To complex, such as SSc, rheumatoid arthritis (RA), pericarditis or interstitial lung disease (ILD). In some embodiments, described herein is a method for detecting anti-Th/To antibodies in a subject who is suspected of having SSc, RA, pericarditis, or ILD. In some embodiments, the subject is suspected of having SSc. In some particular embodiments, the subject is suspected of having IcSSc, a subtype of SSc.

Antinuclear antibodies are valuable biomarkers in the diagnosis of SSc (Mahler M et al, Ann N Y Acad Sci 1 183 :267-87 (2010)). However, ANA HEp-2 is not specific for a particular SARD. Confirmatory testing with molecular target, such as autoantibodies against Th/To complex, is essential to confirm positive ANA result or to more accurately character SSc patients. ANA test can sometimes fail to diagnose a subject with SSc, and the subject with SSc can have a negative ANA result. This subject can have an anti-Th/To autoantibody that can be detected by a method described herein . Additionally, a skilled artisan understands that patients are heterogeneous with respect to the biomarker profiles detectable in their blood, and that biomarker profiling can provide more accurate diagnosis or prognosis of SSc. An additional biomarker for SSc can include any biomarker known in the art. The additional biomarker can include an anti-topoisomerase I antibody, an anti-RNA polymerase III antibody, or an anti- centromere (CENP) antibody. It further includes an anti-PM/Sci complex (exosome) antibody, or an anti-U3RNP/fibrillarin antibody.

In some embodiments, the subject has a negative ANA result. In one aspect, the subject suspected of having SSc is positive for at least one autoantibody selected from the group consisting of an anti-topoisomerase I (topo-I) antibody, an anti-centromere (CENP) antibody and an anti-RNA polymerase III (RNAP) antibody. In yet another aspect, the subject suspected of having SSc is positive for an anti-PM/Scl complex (exosome) antibody or an anti-U3RNP/fibrillarin antibody. In another aspect, the subject suspected of having SSc is negative for at least one autoantibody selected from the group consisting of an anti-topoisomerase I (topo-I) antibody, an anti-centromere (CENP) antibody and an anti-R A polymerase III (R AP) antibody. In yet another aspect, the subject suspected of having SSc is negative for an anti-PM/Scl complex (exosome) antibody or an anti-U3R P/fibrillarin antibody.

In some embodiments, detecting the presence or absence of the anti- Th/To antibody-purified peptide complex includes establishing a level of the anti-Th/To antibody in the sample. In other embodiments, detecting the presence or absence of the anti-Th/To antibody-peptide complex includes comparing the level of anti-Th/To antibody in the sample from the subject to a control level of anti-Th/To antibody in a sample from a healthy control individual, wherein an increase in the anti-Th/To antibody level in the sample compared to the control level indicates that the subject has SSc. In one aspect, the SSc is IcSSc.

As stated above, a skilled artisan understands that the determination of comprehensive biomarker profiles for SSc patients will facilitate the identification of SSc patient subpopulations, aid in the diagnosis of SSc disease subtypes, and aid in the prognostication of disease progression and treatment outcomes for specific SSc disease subtypes. For example, Compared with the anti-CENP positive patients, anti-Th/To IcSSc patients have more subtle cutaneous, vascular, and gastrointestinal involvement, but more often have certain features seen in diffuse SSc, such as pulmonary fibrosis and SSc renal crisis, as well as reduced survival compared to anti-CENP positive patients (Mitri GM et al, Arthritis Rheum 48:203-9(2003)). Like other SSc related autoantibodies, in patients with Raynaud's phenomenon anti-Th/To antibodies are risk factors that are predictive of emerging SSc (Koenig M et al, Arthritis Rheum 58:3902-12(2008)). Anti-Th/To positive patients demonstrated earlier development of nailfold capillary microscopy abnormalities than anti-CENP positive patients (Koenig M et al, Arthritis Rheum 58:3902-12(2008)). Anti-Th/To positive patients were reported to be younger and more frequently male compared to anti-CENP positive patients (Ceribelli A et al, J Rheumatol 37:2071-5(2010)). It has been reported that the prevalence of anti-Th/To antibodies might be higher in Caucasian Americans compared to African and Latin Americans (Krzyszczak ME et al, Clin Rheumatol 30: 1333-9 (2011)).

In some aspects, described herein is a method of diagnosing SSc in a human subject suspected of having SSc, including: a) contacting a sample from the subject with a purified peptide having at least seven contiguous amino acids of an epitope derived from a subunit of a Th/To complex, wherein the epitope has an amino acid sequence selected from the group consisting of SEQ ID NOS: 14, 15, 21, 35, 36, 49, 53, 60-62. 65, 66, 71, 72, and 77-97, or a variant thereof, to form a complex between an anti-Th/To antibody and the purified peptide, and b) detecting the presence or absence of the anti-Th/To antibody-purified peptide complex in the sample, wherein the presence of the anti-Th/To antibody-purified peptide complex in the sample indicates that the subject has SSc; the anti-Th/To antibody can be an anti-Rpp25 antibody, an anti-Rpp38 antibody, an anti-hPop1 antibody, or any combination thereof. In some aspects , the at least seven contiguous amino acids can be at least eight, night, ten, eleven, twelve, thirteen, fourteen, fifteen, sixteen, seventeen, eighteen, nineteen, or twenty amino acids.

In one aspect, the anti-Th/To antibody is an anti-Rpp25 antibody; in one aspect, the anti-Th/To antibody is an anti-Rpp38 antibody; in one aspect, the anti-Th/To antibody is an anti-hPop1 antibody; in one aspect, the anti-Th/To antibody is an anti-Rpp25 antibody and an anti-Rpp38 antibody; in one aspect, the anti-Th/To antibody is an anti-Rpp25 antibody and an anti-hPop1 antibody; in one aspect, the anti-Th/To antibody is an anti- hPop1 antibody and an anti-Rpp38 antibody; in another aspect, the anti-Th/To antibody is an anti-Rpp25 antibody, an anti-Rpp38 antibody, and an anti-hPop1 antibody.

In some aspects, the purified peptide used in the method of diagnosing SSc has an amino acid sequence selected from the group consisting of SEQ ID NOS: 4-80 and 98-104. In one aspect, the purified peptide has the amino acid sequence of SEQ ID NO: 25. In another aspect, the purified peptide has the amino acid sequence of SEQ ID NO: 102.

The subject can be a healthly subject. In some embodiments, the health subject has never suffered from a certain disease. In some embodiments, the healthy subject was previously diseased. In some embodiments, the healthy subject is undergoing a routine medical checkup. In some embodiments, the healthy subject is a member of a control group in a clinical trial. In some embodiments, the healthy subject is at risk of contracting a disease, as determined by the presence of certain risk factors that are well known in the art. Such risk factors include, without limitation, a genetic predisposition, a personal disease history, a familial disease history, a lifestyle factor, an environmental factor, a diagnostic indicator, and the like.

In some embodiments, the subject is asymptomatic. An asymptomatic subject include a healthy subject who has essentially no risk or only a low risk of developing SSc *(e.g.,* there is a less than 10%, less than 5%, less than 3%, or less than 1% probability that the asymptomatic patient will develop SSc over the following five year period). An asymptomatic subject further includes a healthy subject who has a high risk of developing SSc *(e.g.,* there is a greater than 50%, greater than 70%, greater than 90%, or greater than 95% probability that the asymptomatic patient will develop SSc over the following five year period). An asymptomatic subject further includes a diseased subject, who may display mild early diagnostic indicators of SSc, but who is otherwise disease or complaint free.

In some embodiments, the subject has SSc. In some embodiments, the subject is suspected of having SSc. In some embodiments, the subject has a symptom of Raynaud's phenomenon (for example, color change of fingers or toes when exposed to cold), thickening of skin, swelling of the hands, or general pain. In some embodiments, the subject has an early symptom of lupus, rheumatoid arthritis, dermatomyositis.

In some embodiments, the subject is at risk of developing SSc. In some embodiments, the subject has a genetic predisposition for developing SSc or a family history of SSc. In some embodiments, the subject is exposed to certain lifestyle factors promoting the development of SSc or the subject shows clinical disease manifestations of SSc. In some embodiments, the subject is a patient who is receiving a clinical workup to diagnose SSc or to assess the risk of developing SSc.

In some embodiments, the subject is treatment naïve. In some embodiments, the subject is undergoing treatments for SSc *(e.g.,* drug treatments). In some embodiments, the subject is in remission. In some embodiments, the remission is drug-induced. In some embodiments, the remission is drug-free.

In one embodiment, the subject has a negative ANA result. In another embodiment, the subject suspected of having SSc is positive for at least one autoantibody selected from the group consisting of an anti-topoisomerase I (topo-I) antibody, an anti-centromere (CENP) antibody and an anti-RNA polymerase III (RNAP) antibody. In yet another embodiment, the subject suspected of having SSc is positive for an anti-PM/Scl complex (exosome) antibodoy or an anti-U3RNP/fibrillarin antibody. In another embodiment, the subject suspected of having SSc is negative for at least one autoantibody selected from the group consisting of an anti-topoisomerase I (topo-I) antibody, an anti-centromere (CENP) antibody and an anti-RNA polymerase III (RNAP) antibody. In yet another embodiment, the subject suspected of having SSc is negative for an anti-PM/Scl complex (exosome) antibody or an anti-U3RNP/fibrillarin antibody.

In other aspects, described herein is a method of diagnosing SSc in a human subject suspected of having SSc, the SSc is IcSSc.

In some aspects, described herein is a method of determining the prognosis of SSc in a human subject, including a) contacting a sample from the subject with the purified peptide having at least seven contiguous amino acids of an epitope derived from a subunit of a Th/To complex, wherein the epitope has an amino acid sequence selected from the group consisting of SEQ ID NOS: 14, 15, 21, 35, 36, 49, 53, 60-62. 65, 66, 71, 72, and 77-97, or a variant thereof, to form a complex between an anti-Th/To antibody and the purified peptide, and b) detecting the presence or absence of the anti-Th/To antibody-purified peptide complex in the sample, wherein the presence of the anti-Th/To antibody-purified peptide complex in the sample indicates the course of SSc progression in the human subject; and the anti-Th/To antibody can be an anti-Rpp25 antibody, an anti-Rpp38 antibody, an anti-hPopl antibody, or any combination thereof. The at least seven contiguous amino acids can be at least eight, night, ten, eleven, twelve, thirteen, fourteen, fifteen, sixteen, seventeen, eighteen, nineteen, or twenty amino acids.

In one aspect, the anti-Th/To antibody is an anti-Rpp25 antibody; in one aspect, the anti-Th/To antibody is an anti-Rpp38 antibody; in one aspect, the anti-Th/To antibody is an anti-hPopl antibody; in one aspect, the anti-Th/To antibody is an anti-Rpp25 antibody and an anti-Rpp38 antibody; in one aspect, the anti-Th/To antibody is an anti-Rpp25 antibody and an anti-hPopl antibody; in one aspect, the anti-Th/To antibody is an anti-hPopl antibody and an anti-Rpp38 antibody; in another aspect, the anti-Th/To antibody is an anti-Rpp25 antibody, an anti-Rpp38 antibody, and an anti-hPopl antibody.

In some aspects, the purified peptide used in the method of determining the prognosis of SSc has an amino acid sequence selected from the group consisting of SEQ ID NOS: 4-80 and 98-104. In one aspect, the purified peptide has the amino acid sequence of SEQ ID NO: 25. In other aspect, the purified peptide has the amino acid sequence of SEQ ID NO: 102.

In some aspects, described herein is a method of determining the prognosis of SSc in a human subject, wherein the human subject is an asymptomatic subject suspected to be at risk of developing SSc.

As used herein, the term "at risk" refers to an increased likelihood that a subject will develop a certain disease condition or clinical symptoms of disease in the future. For example, a subject who is "at risk of developing SSc" or "at risk of developing clinical symptoms of SSc" is more likely in the future to develop SSc or clinical symptoms of SSc than the median or average subject in a given population. A subject who is "at risk" of developing a disease condition in the future does not already suffer from this condition. A subject who is "at risk" of developing a disease condition can display certain biomarkers, such as an elevated level of anti-Th/To antibodies, that indicate an increased likelihood that the subject will develop a certain disease condition (e.g., SSc) or clinical symptoms of a certain disease condition (e.g., skin thickening and tightness, shortness of breath, reflux, fatigue, etc.).

In some aspects, described herein is a method of determining the prognosis of SSc in a human subject , wherein the human subject is an asymptomatic subject suspected to be at risk of developing SSc, and the presence of the anti-Th/To antibody-purified peptide complex in the sample indicates that the human subject is at a greater risk of developing SSc than the absence of the anti-Th/To antibody-purified peptide complex.

In some aspects, described herein is a method of determining the prognosis of SSc in a human subject, wherein the human subject is a SSc patient having a clinical symptom of SSc. In some aspects, the presence of the anti-Th/To antibody-purified peptide complex in the sample predicts a more severe clinical course of SSc disease progression than the absence of the anti-Th/To antibody-purified peptide complex.

In some aspects, described herein is a method of determining the prognosis of SSc in a human subject, wherein detecting the presence or absence of the anti- Th/To antibody-purified peptide complex in the sample includes determining the level of anti- Th/To antibodies in the sample. In one aspect, a higher level of anti-Th/To antibodies in the sample indicate a higher risk that an asymptomatic subject will develop SSc than a lower level of anti-Th/To antibodies. In another aspect, a higher level of anti-Th/To antibodies in the sample indicate a more severe course of future disease progression in a SSc patient than a lower level of anti-Th/To antibodies.

In some aspects, described herein is a method of determining the prognosis of SSc in a human subject, wherein the SSc is limited IcSSc.

In some aspects, described herein is a method of monitoring the efficacy of a SSc treatment in a SSc patient, including: a) contacting two or more samples obtained from the patient at a first and at least one subsequent time point throughout the course of the SSc treatment with the purified peptide described herein to form a complex between the anti-Th/To antibodies and the purified peptide; b) determining the levels of anti-Th/To antibodies in the two or more samples, and c) comparing the levels of anti-Th/To antibodies in the two or more samples, where a decreased level of anti-Th/To antibodies in a sample obtained at a subsequent time point relative to the level of anti-Th/To antibodies in the sample obtained at the first time point indicate that the SSc treatment is efficacious; the anti-Th/To antibody can be an anti-Rpp25 antibody, an anti-Rpp38 antibody, an anti-hPopl antibody, or any combination thereof.

In some aspects, one or more samples were obtained at the beginning of the course of the SSc treatment and one or more samples were obtained at later time points throughout the course of the SSc treatment. In some aspects, the subsequent time points are 2 or more, 3 or more, 4 or more, 5 or more, 6 or more, 7 or more, 8 or more, 9 or more, 10 or more, 15 or more, 20 or more, 25 or more or 30 or more time points.

In some aspects, the SSc treatments include drug treatments. In some aspects, the drug treatment includes an immunosuppressive medication, such as corticosteroid, methotrexate, or mycophenalate mofetil. In some aspects, the drug treatment include a FDA- approved SSc drug, including an experimental SSc drug in clinical development. In some embodiments, the SSc treatment includes treatments with a combination of two or more drugs.

In some aspects, the method further includes adjusting the treatment if the treatment is determined to be not efficacious. Adjusting the treatment can include, for example, adjusting the dose of a drug treatment, increasing the frequency of a drug treatment, treating with a different drug or combination of drugs, ending the treatment.

In some aspects, the method further includes repeating a treatment if the treatment is determined to be efficacious.

In some aspects, the level of anti-Th/To antibodies in the samples obtained at the first time point is decreased by more than 10%, more than 20%, more than 30%, more than 40%,
more than 50%, more than 60%, more than 70%, more than 80%, more than 90%, more than 95%), or more than 99% in a subsequence time point.

In other aspects, described herein is a method of monitoring the efficacy of a SSc treatment in a SSc patient, wherein the SSc is IcSSc.

In some aspects, described herein is a kit for detecting an anti- Th/To antibody in a sample from a subject or for diagnosing an autoimmune disease, having a purified peptide described herein and an ancillary reagent. The anti-Th/To antibody can be an anti-Rpp25 antibody, an anti-Rpp38 antibody, an hPop1 antibody, or any combination thereof. In some aspects, the purified peptide has at least seven contiguous amino acids of an epitope derived from a subunit of a Th/To complex, wherein the epitope has an amino acid sequence selected from the group consisting of SEQ ID NOS: 14, 15, 21 , 35, 36, 49, 53, 60-62. 65, 66, 71 , 72, and 77-97 or a variant thereof. The at least seven contiguous amino acids can be at least eight, night, ten, eleven, twelve, thirteen, fourteen, fifteen, sixteen, seventeen, eighteen, nineteen, or twenty amino acids.

In some aspects, the purified peptide has an amino acid sequence selected from the group consisting of SEQ ID NOS: 4-80 and 98-104. In some aspects, the purified peptide has the amino acid sequence of SEQ ID NO: 25. In other aspects, the purified peptide has the amino acid sequence of SEQ ID NO: 102.

The kit of this disclosure can include any immobilization reagent known in the art, including covalent and non-covalent immobilization reagents. Covalent immobilization reagents can include any chemical or biological reagent that can be used to covalently immobilize a peptide of this disclosure on a surface. Covalent immobilization reagents can include, for example, a carboxyl-to-amine reactive group (e.g. , carbodiimides such as EDC or DCC), an amine reactive group (e.g., N-hydroxysuccinimide (NHS) esters, imidoesters), a sulfhydryl- reactive crosslinker (e.g., maleimides, haloacetyls, pyridyl disulfides), a carbonyl-reactive crosslinker groups (e.g., hydrazides, alkoxyamines), a photoreactive crosslinker (e.g., aryl azides, dizirines), or a chemoselective ligation group (e.g., a Staudinger reaction pair). Non-covalent immobilization reagents include any chemical or biological reagent that can be used to immobilize a peptide of this disclosure non-covalently on a surface, such as affinity tags (e.g., biotin) or capture reagents (e.g., streptavidin or anti-tag antibodies, such as anti-His6("Hise" disclosed as SEQ ID NO: 1 13) or anti-Myc antibodies).

The kits of this disclosure can include combinations of immobilization reagents. Such combinations include, for example, EDC and NHS, which can be used, for example, to immobilize a protein of this disclosure on a surface, such as a carboxylated dextrane matrix (*e.g*., on a BIAcore^{™} CM5 chip or a dextrane-based bead). Combinations of immobilization reagents can be stored as premixed reagent combinations or with one or more immobilization reagents of the combination being stored separately from other immobilization reagents.

In some embodiments, the ancillary reagent is selected from the group consisting of a secondary antibody, a detection reagent, an immobilization buffer, a blocking buffer, a washing buffer, and a detection buffer. In some aspects, the secondary antibody is selected from an anti-human IgA antibody, anti-human IgD antibody, anti-human IgE antibody, anti-human IgG antibody, and anti-human IgM antibody. In some aspects, the detection reagent contains a fluorescent detection reagent or a luminescent detection reagent. In some other aspects, the luminescent detection reagent contains luminol or luciferin.

A large selection of washing buffers are known in the art, such as tris(hydroxymethyl)aminomethane (Tris)-based buffers (*e.g*., Tris-buffered saline, TBS) or phosphate buffers (*e.g*., phosphate-buffered saline, PBS). Washing buffers can include detergents, such as ionic or non-ionic detergents. In some embodiments, the washing buffer is a PBS buffer (*e.g.,* about pH 7.4) including Tween^{®}20 (*e.g.,* about 0.05% Tween^{®}20). In some embodiments, the washing buffer is the BIO-FLASH^{™} Special Wash Solution (INOVA Diagnostics, Inc., San Diego, CA).

Any dilution buffer known in the art can be included in a kit of this disclosure. Dilution buffers can include a carrier protein (*e.g.*, bovine serum albumin, BSA) and a detergent (*e.g.,* Tween^{®}20). In some embodiments, the dilution buffer is PBS (*e.g.,* about pH 7.4) including BSA (*e.g.,* about 1% BSA) and Tween^{®}20 (*e.g.,* about 0.05% Tween^{®}20).

Secondary antibodies can include, for example, an anti-human IgA antibody, an anti-human IgD antibody, an anti-human IgE antibody, an anti-human IgG antibody, or an anti-human IgM antibody. In some embodiments, the secondary antibodies are anti-bovine antibodies. Secondary detection antibodies can be monoclonal or polyclonal antibodies. Secondary antibodies can be derived from any mammalian organism, including mice, rats, hamsters, goats, camels, chicken, rabbit, and others. Secondary antibodies can be conjugated to enzymes (*e.g*., horseradish peroxidase (HRP), alkaline phosphatase (AP), luciferase, and the like) or dyes (*e.g*., colorimetric dyes, fluorescent dyes, fluorescence resonance energy transfer (FRET)-dyes, time-resolved (TR)-FRET dyes, and the like). In some embodiments, the secondary antibody is a polyclonal rabbit-anti-human IgG antibody, which is HRP-conjugated.

In some embodiments, the detection reagent is a colorimetric detection reagent, a fluorescent detection reagent, or a chemiluminescent detection reagent. In some embodiments, the colorimetric detection reagent includes PNPP (p-nitrophenyl phosphate), ABTS (2,2'-azino-bis(3-ethylbenzothiazoline-6-sulphonic acid)) or OPD (o-phenylenediamine). In some embodiments, the fluorescent detection reagent includes QuantaBlu^{™} or QuantaRed^{™} (Thermo Scientific, Waltham, MA). In some embodiments, the luminescent detection reagent includes luminol or luciferin. In some embodiments, the detection reagent includes a trigger (*e.g*., H₂O₂) and a tracer (*e*.*g*., isoluminol-conjugate). In some embodiments, the detection reagent includes one or more BIO-FLASH^{™} Trigger solutions (INOVA Diagnostics, Inc., San Diego, CA).

Any detection buffer known in the art can be included in a kit of this disclosure. In some embodiments the detection buffer is a citrate-phosphate buffer (*e.g*., about pH 4.2).

Any stop solution known in the art can be included in a kit of this disclosure. The stop solutions of this disclosure terminate or delay the further development of the detection reagent and corresponding assay signals. Stop solutions can include, for example, low-pH buffers (*e.g.,* glycine-buffer, pH 2.0), chaotrophic agents (*e.g.,* guanidinium chloride, sodium-dodecylsulfate (SDS)) or reducing agents (*e.g*., dithiothreitol, β-mecaptoethanol), or the like.

In some embodiments, the kit of this disclosure includes a cleaning reagent for an automated assay system. An automated assay system can include systems by any manufacturer. In some embodiments, the automated assay systems include, for example, the BIO-FLASH^{™}, the BEST 2000^{™}, the DS2^{™}, the ELx50 WASHER, the ELx800 WASHER, the ELx800 READER, and the Autoblot S20^{™} (INOVA Diagnostics, Inc., San Diego, CA). A cleaning reagent can include any cleaning reagent known in the art. In some embodiments, the cleaning reagent is the cleaning reagent recommended by the manufacturer of the automated assay system. In some embodiments, the cleaning reagent can be the BIO-FLASH^{™} System Rinse or the BIO-FLASH^{™} System Cleaning solutions (INOVA Diagnostics, Inc., San Diego, CA).

In some embodiments, the kit further includes a solid support. The solid support can include any support known in the art on which a protein of this disclosure can be immobilized. In some embodiments, solid the solid substrates are microtiter well plates, slides (*e.g.,* glass slides), chips (e.g., protein chips, biosensor chips, such as Biacore chips), microfluidic cartridges, cuvettes, beads (e.g., magnetic beads) or resins.

In some embodiments, the kit of this disclosure includes a microtiter plate. In one aspect, the microtiter plate is a 96-well plate, a 384-well plate, or a 1536-well plate. In another aspect, the purified peptide is immobilized in one or more wells of the microtiter plate. In some embodiments, the microtiter plate is a Nunc Maxisorp^{®} plate (e.g., Fisher Scientific, Hampton, NH, cat# 430341).

In some embodiments, the kit of this disclosure includes a cuvette. In some embodiments, the cuvette is a BIO-FLASH^{™} Cuvette (INOVA Diagnostics, Inc., San Diego, CA).

In some embodiments, the kit of this disclosure includes one or more additional consumables. In some embodiments, the consumable is a sample cup (e.g., 1ml, 5ml, 10ml, 25ml, or 50ml sample cup) or a screw cap. In some embodiments, the sample cup is a Falcon^{™} Tube (BD Biosciences, San Jose, CA) or the like. In some embodiments, the sample cup is a BIO-FLASHTM Sample Cup (INOVA Diagnostics, Inc., San Diego, CA). In some embodiments, the screw cap is a BIO-FLASHTM Screw Cap (INOVA Diagnostics, Inc., San Diego, CA).

In some other embodiments, the kit of this disclosure includes instruction for using the subunits of the kit for detecting anti- Th/To antibodies in the sample from the subject or for diagnosing the autoimmune disease.

The kit of this disclosure can be tailored to specific assay technologies. In some embodiments, the kit is an ELISA kit, Dot Blot kit, chemiluminescence immunoassay (CIA) kit or multiplex kit. In some embodiments, the ELSA kit can include a washing buffer, a sample diluents, a secondary antibody-enzyme conjugate, a detection reagent and a stop solution. In some embodiments, the Dot Blot kit includes a washing buffer, a sample diluents, a secondary antibody-enzyme conjugate, a detection reagent, and a stop solution. In some embodiments, the CIA kit includes a washing buffer, a sample diluent, a tracer (e.g., isoluminol-conjugate) and a trigger (e.g., H202). In some embodiments, the multiplex kit includes a washing buffer, a sample diluents and a secondary antibody-enzyme conjugate.

Described herein is a kit for diagnosing an autoimmune disease, the autoimmune disease being SSc, rheumatoid arthritis (RA), pericarditis
or interstitial lung disease (ILD). In some aspects, the SSc is IcSSc. In some aspects, the kit is used to diagnose SSc patients, to differentiate SSc patients subpopulations (for example, to differentiate anti-Th/To positive and anti-Th/To negative patients), to prognosticate disease progression in SSc patients (for example, to predict a more severe disease progression in anti- Th/To positive relative to anti-Th/To negative patients), to monitor the efficacy of SSc treatment or to predict treatment outcomes. In some aspects, the SSc treatment can include drug treatment. In some aspects, the drug treatment includes an immunosuppressive medication, such as corticosteroid, methotrexate, or mycophenalate mofetil. In some aspects, the drug is a FDA-approved drug, including an experimental SSc drug in clinical development. In some aspects, the kit is used as companion diagnostics for SSc treatments. In some aspects, the kit of this disclosure is used to select a patient specific SSc drug treatments.

In some aspects, the kit has a packaging that includes a label indicating the kit is used for diagnosis, prognosis or monitoring of SSc, RA, ILD or IcSSc. In some aspects, the kit is used as companion diagnostics for SSc treatments. In some other aspects, the packaging has a label indicates that the kit is used with a SSc drug. In some aspects, the kit is used to select a patient specific SSc drug treatments.

In some aspects, the packaging of the kit includes an FDA-approved label. FDA approved labels can include notification of an FDA-approved use and instructions. In some aspects, the kit is labeled for Research Use Only (RUO) or for Investigational Use Only (IUO). In some aspects, the kit is labeled for In Vitro Diagnostic Use (IVD). In some aspects, the kit is labeled in accordance with Title 21, Code of Federal Regulations, Section 809, Subpart B (21 CFR 89, Subpart B). In some aspects, the RUO, IUO, or IVD labels of the kit describes the use of the kit for the diagnosis of SSc. In some aspects, the RUO, IUO, or IVD labels of the kit describes the use of the kit for the diagnosis of a SSc subtype, for example, LcSSc. In some aspects, the RUO, IUO, or IVD labels of the kit describes the use of the kit for the prognostication of SSc. In some aspects, the kit is labeled as IVD companion diagnostic device for use with a SSc drug.

The following examples are intended to illustrate but not limit the present invention.

### EXAMPLE I

### Identification of Novel B-cell Epitopes on Three Subunits of the Th/To Complexes (Rpp25, Rpp38 and hPopl)

This example illustrates the identification of novel B-cell epitopes on three subunits of the Th/To complexes (Rpp25, Rpp38, and hPopl) as immunological targets of circulating autoantibides in the serum of human patients with SSc, a type of SARD.

### Discovery of linear epitopes using high density solid phase peptide array

**Sera:** Three pools of sera were generated based on three individual samples each. Two pools were based on samples with anti-Th/To antibodies (Th/To pool 1 and 2) and one pool contained three sera from systemic lupus erythematosus (SLE) samples. A panel of SSc patient samples with a nucleolar pattern by indirect immunofluorescence was used to verify the results using the biotinylated soluble peptides.

**Measurement of autoantibodies:** ANA were detected by indirect immunofluorescence (IIF) performed on HEp-2 substrate (HEp-2000; ImmunoConcepts, Sacramento, CA, USA) that included fluorescein-conjugated goat antibodies to human IgG (H+L). IIF patterns were detected at serum screening dilutions of 1:160 and 1:640 on a Zeiss Axioskop 2 plus (Carl Zeiss, Jena, Germany) fitted with a 100-watt USHIO super-high-pressure mercury lamp (Ushio, Steinhöring, Germany) by two experienced technologists with more than 7 years of experience.

**Epitope mapping using solid phase peptides:** Antigens Rpp25, Rpp38 and hpop1 were translated into peptides of 15 amino acids with peptide-peptide overlaps of 14 amino acids (Rpp25 and Rpp38) or with peptide-peptide overlaps of 13 amino acids (hpopl) resulting in 1,041 different peptides printed in duplicate (2,082 peptide spots in all) using solid phase peptide arrays (Pepperprint, Heidelberg, Germany) (Mierau R et al, Arthritis Res Ther 13:R172(2011); Beyer M et al, Methods Mol Biol 2009;570:309-16 (2009); Nesterov A et al, Methods Mol Biol 669:109-24(2010)). The corresponding peptide microarrays were further framed by Flag and HA control peptides (100 spots each). Three serum pools were used: Th/To Pool 1, Th/To Pool 2 and SLE Pool 3. Sample pools were diluted in three serum dilutions (1:1000, 1:250 and 1:100) in incubation buffer (PBS, pH 7.4 with 0.05% Tween 20 and 10% Rockland blocking buffer MB-070) and incubation for 16 h at 4°C and shaking at 500 rpm. Unbound antibodies were removed by washing the arrays with PBS, pH 7.4 with 0.05% Tween 20 (2x1 min after each assay). Pre-staining of one of the peptide arrays was done with the F(ab')2 goat anti-human IgG(H+L) conj. DyLight680 antibody at a dilution of 1:5000 to investigate background interactions with the antigen-derived peptides that could interfere with the main assays. Subsequent incubation of the peptide microarrays with human sera Th/To Pool 1, Th/To Pool 2 and SLE Pool 3 at dilutions of 1:1000, 1:250 and 1:100 in incubation buffer was followed by staining with the secondary antibody and read-out at a scanning intensity of 7 (red). HA and Flag control peptides framing the peptide arrays were finally stained with monoclonal anti-HA (12CA5)-DyLight680, monoclonal anti-FLAG(M2)-DyLight800; staining in incubation buffer for 1 h at room temperature and a dilution of 1:1000 as internal quality control to confirm the assay quality and the peptide microarray integrity (scanning intensities red/green: 7/7).

Quantification of spot intensities and peptide annotation were done with LI-COR Odyssey Imaging System (scanning offset 1 mm, resolution 21 µm, scanning intensities in green/red of 7/7) and PepSlide^{®} Analyzer. A software algorithm breaks down fluorescence intensities of each spot into raw, foreground and background signal, and calculates the standard deviation of foreground median intensities. Based on averaged foreground median intensities, intensity maps were generated and binders in the peptide maps highlighted by an intensity color code with red for high and white for low spot intensities.

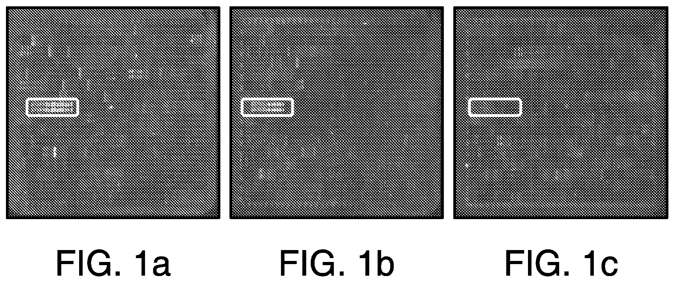

**Results:** Two pools of sera with anti-Th/To antibodies and one control pool were used to identify epitopes on Rpp25, Rpp38 and hPop1. The highest signals were found for a long stretch of peptides from the Rpp38 antigen (Fig. 1). These peptides reacted with both Th/To serum pools, but not with the control pool indicating specific reactivity. Moreover, the assays gave rise to some additional but weaker epitope-like spot patterns in array areas covered by antigens Rpp38 and hPop1.

Next, all peptides that yielded at least 200 units (at 1:250 dilution) with one of the Th/To serum pools, but not with the SLE pool were selected for further analyses. For Rpp25 one peptide was positive for both pools, ten samples for Th/To pool 1 and seven peptides for Th/To pool 2. Rpp38 displayed a significantly higher number of positive peptides. Seven peptides were positive for both pools, six samples for Th/To pool 1 and 24 peptides for Th/To pool 2. Lastly, on hPop1 two peptides were positive for both pools, 16 samples for Th/To pool 1 and three peptides for Th/To pool 2. Number and percent of positive peptides (>200 units) and highest signals are summarized in Table 1. The sequences of positive peptides are summarized in Table 2.

**Table 1: Positive Peptides (>200 units) and Max Reactivity recognized by Th/To positive pools (dilution 1:250)**

| **Antigen** | **No. peptides >200** | **No. peptides tested** | **Epitope regions No.** | **% peptide positive** | **Max reactivity** |
|---|---|---|---|---|---|
| Rpp25 | 18 | 186 | 7 | 9.7% | 1531 |
| Rpp38 | 37 | 270 | 12 | 13.7% | 7158 |
| hPop1 | 21 | 506 | 16 | 4.2% | 899 |

**Table 2: Positive Peptides (>200 units) (dilution 1:250)**

| **Rpp25** | |
|---|---|
| **SEQ ID NO:** | **Amino Acid Sequence** |
| 4 | ATASMAQPATRAIVF |
| 5 | TASMAQPATRAIVFS |
| 6 | TQGQTPGEPAASLSV |
| 7 | EPAASLSVLKNVPGL |
| 8 | VPGLAILLSKDALDP |
| 9 | PGLAILLSKDALDPR |
| 10 | GLAILLSKDALDPRQ |
| 11 | AILLSKDALDPRQPG |
| 12 | LLSKDALDPRQPGYQ |
| 13 | KDALDPRQPGYQPPN |
| 14 | EPGVADEDQTAGSGS |
| 15 | GPGSGPFADLAPGAV |
| 16 | AFATASMAQPATRAI |
| 17 | FATASMAQPATRAIV |
| 18 | PTQGQTPGEPAASLS |
| 19 | AASLSVLKNVPGLAI |
| 20 | KNVPGLAILLSKDAL |
| 21 | RQPGYQPPNPHPGPS |

| **Rpp38** | |
|---|---|
| **SEQ ID NO:** | **Amino Acid Sequence** |
| 22 | SQDRELLDTSFEDLS |
| 23 | QDRELLDTSFEDLSK |
| 24 | DRELLDTSFEDLSKP |
| 25 | RELLDTSFEDLSKPK |
| 26 | ELLDTSFEDLSKPKR |
| 27 | LLDTSFEDLSKPKRK |
| 28 | LDTSFEDLSKPKRKL |
| 29 | PYIIRWSALESEDMH |
| 30 | SALESEDMHFILQTL |
| 31 | ESEDMHFILQTLEDR |
| 32 | TDAKQQVSGWTPAHV |
| 33 | PAHVRKQLAIGVNEV |
| 34 | AHVRKQLAIGVNEVT |
| 35 | KTSLNNPYIIRWSAL |
| 36 | MHFILQTLEDRLKAI |
| 37 | IEDKKKKNKTPFLKK |
| 38 | EDKKKKNKTPFLKKE |
| 39 | DKKKKNKTPFLKKES |
| 40 | KKKKNKTPFLKKESR |
| 41 | KKNKTPFLKKESREK |
| 42 | PFLKKESREKCSIAV |
| 43 | ISENLKEKKTDAKQQ |
| 44 | SENLKEKKTDAKQQV |
| 45 | ENLKEKKTDAKQQVS |
| 46 | KEKKTDAKQQVSGWT |
| 47 | KKTDAKQQVSGWTPA |
| 48 | VSGWTP AHVRKQLAI |
| 49 | RKQLAIGVNEVTRAL |
| 50 | ERIAPVIGLKCVLAL |
| 51 | RIAPVIGLKCVLALA |
| 52 | IAPVIGLKCVLALAF |
| 53 | FKKNTTDFVDEVRAI |
| 54 | DTSFEDLSKPKRKLA |
| 55 | TSFEDLSKPKRKLAD |
| 56 | SFEDLSKPKRKLADG |
| 57 | FEDLSKPKRKLADGR |
| 58 | KKLIPNPNKIRKPPK |
| 59 | KIRKPPKSKKATPKQ |

| **hPop1** | |
|---|---|
| **SEQ ID NO:** | **Amino Acid Sequence** |
| 60 | ISDLTMEMNRFRLIG |
| 61 | GDPRINLPQKKSKAL |
| 62 | EISAMLKAVTQKSSN |
| 63 | ILKALSGMCNIDTGL |
| 64 | TGLTFAAVHCLSGKR |
| 65 | ADPLPTPSQEKSQTE |
| 66 | TRDPCLPYSWISPTT |
| 67 | TGIIISDLTMEMNRF |
| 68 | TMEMNRFRLIGPLSH |
| 69 | QLLLEGVPVECTHSF |
| 70 | EGVPVECTHSFIWNQ |
| 71 | KRSPNVPGDFPDCPA |
| 72 | EPHTMICVPAKEDFL |
| 73 | WHYCGPQESKHSDPF |
| 74 | GPQESKHSDPFRSKI |
| 75 | SDGPAGEEPVAGQEA |
| 76 | PVAGQEALTLGLWSG |
| 77 | TGLLDMLSSQPAAQR |
| 78 | ADRGVKHHSGGEKPF |
| 79 | NAKPIKKIIGDGTRD |
| 80 | REACLSILGHFPRAL |

Figure 2 demonstrates the linear epitope mapping of reactive peptids from the three proteins subunits of Th/To complex. Positive peptides from Rpp25 are shown in a), from Rpp38 in b) and from hPop1 in c). Different epitope regions are separated by the vertical lines in the figure. The epitopes are also listed below in Table 3.

**Table 3: Epitope regions**

| **Region** | **SEQ ID NO:** | **SEQ ID NO: of 15mer** | **Amino Acid Sequence** |
|---|---|---|---|
| **Rpp25** | | | |
| 1 | 81 | 4, 5 | ATASMAQPATRAIVFS |
| 2 | 82 | 6-13 | TQGQTPGEPAASLSVLKNVPGLAILLSKDALDPRQPGYQPPN |
| 3 | 14 | 14 | EPGVADEDQTAGSGS |
| 4 | 15 | 15 | GPGSGPFADLAPGAV |
| 5 | 83 | 16, 17 | AFATASMAQPATRAIV |
| 6 | 84 | 18-20 | PTQGQTPGEPAASLSVLKNVPGLAILLSKDAL |
| 7 | 21 | 21 | RQPGYQPPNPHPGPS |

| **Rpp38** | | | |
|---|---|---|---|
| 1 | 85 | 22-28 | SQDRELLDTSFEDLSKPKRKL |
| 2 | 86 | 29-31 | PYIIRWSALESEDMHFILQTLEDR |
| 3 | 87 | 32-34 | TDAKQQVSGWTPAHVRKQLAIGVNEVT |
| 4 | 35 | 35 | KTSLNNPYIIRWSAL |
| 5 | 36 | 36 | MHFILQTLEDRLKAI |
| 6 | 88 | 37-42 | IEDKKKKNKTPFLKKESREKCSIAV |
| 7 | 89 | 43-48 | ISENLKEKKTDAKQQVSGWTPAHVRKQLAI |
| 8 | 49 | 49 | RKQLAIGVNEVTRAL |
| 9 | 90 | 50-52 | ERIAPVIGLKCVLALAF |
| 10 | 53 | 53 | FKKNTTDFVDEVRAI |
| 11 | 91 | 54-57 | DTSFEDLSKPKRKLADGR |
| 12 | 92 | 58, 59 | KKLIPNPNKIRKPPKSKKATPKQ |

| **hPop1** | | | |
|---|---|---|---|
| 1 | 60 | 60 | ISDLTMEMNRFRLIG |
| 2 | 61 | 61 | GDPRINLPQKKSKAL |
| 3 | 62 | 62 | EISAMLKAVTQKSSN |
| 4 | 93 | 63, 64 | ILKALSGMCNIDTGLTFAAVHCLSGKR |
| 5 | 65 | 65 | ADPLPTPSQEKSQTE |
| 6 | 66 | 66 | TRDPCLPYSWISPTT |
| 7 | 94 | 67-68 | TGIIISDLTMEMNRFRLIGPLSH |
| 8 | 95 | 69-70 | QLLLEGVPVECTHSFIWNQ |
| 9 | 71 | 71 | KRSPNVPGDFPDCPA |
| 10 | 72 | 72 | EPHTMICVPAKEDFL |
| 11 | 96 | 73, 74 | WHYCGPQESKHSDPFRSKI |
| 12 | 97 | 75, 76 | SDGPAGEEPVAGQEALTLGLWSG |
| 13 | 77 | 77 | TGLLDMLSSQPAAQR |
| 14 | 78 | 78 | ADRGVKHHSGGEKPF |
| 15 | 79 | 79 | NAKPIKKIIGDGTRD |
| 16 | 80 | 80 | REACLSILGHFPRAL |

### Anti-Th/To antibodies to synthetic Th/To derived peptides

**Detection of antibodies to candidate peptide:** Soluble biotinylated peptides were coupled to streptavidine beads and tested on a MagPix instrument (Luminex, Austin, Texas, US).

**Statistical evaluation:** The data was statistically evaluated using the Analyse-it software (Version 1.62; Analyse-it Software, Ltd., Leeds, UK). Chi-square, Spearman's correlation and Cohen's kappa agreement test were carried out to analyze the agreement between portions and p values < 0.05 were considered significant. Receiver-operating characteristics (ROC) analysis was used to analyse the discriminatory ability of different immunoassays. Descriptive statistics were used to summarize the baseline characteristics of the patients. Chi-squared tests, Fisher's exact tests and Mann Whitney U tests were used as appropriate. P values < 0.05 were considered statistically significant. These statistical analyses were performed with SAS v.9.2 (SAS Institute, USA). Clustering illustrates the relationship between different assays, as described by Eisen MB et al, Proc Natl Acad Sci U S A 95:14863-8(1998). Hierarchical clustering was performed using the following criteria: Average linkage clustering, patient correlation uncentered and reactivities centered.

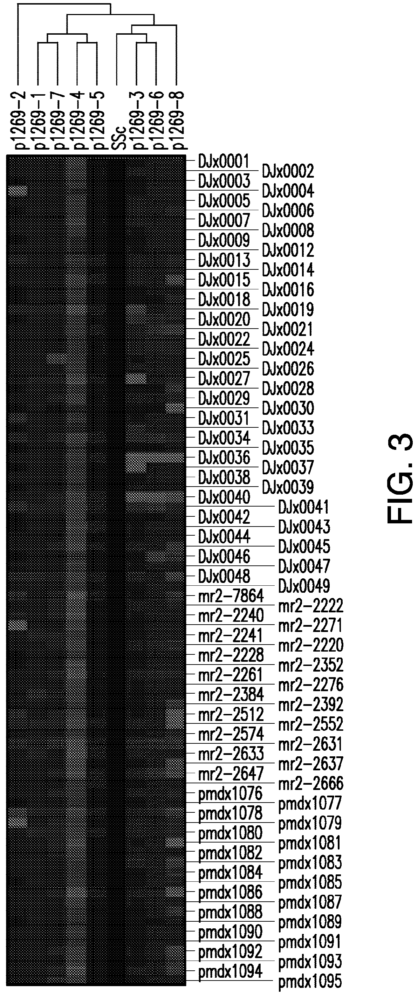

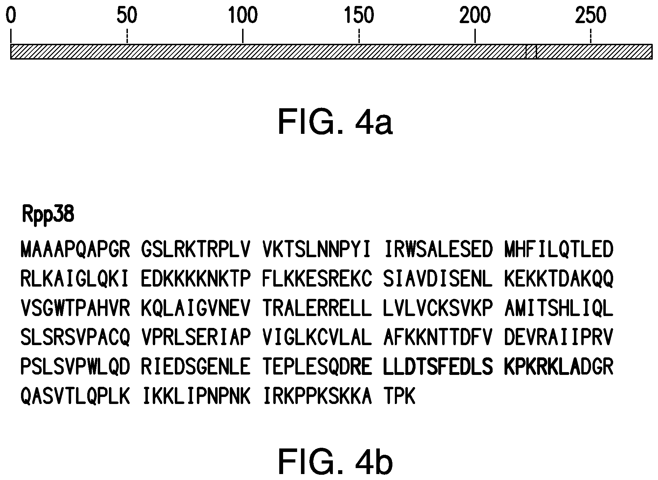

**Results:** A total of eight soluble biotinylated peptides were designed based on the identified sequences and used for further studies. Two peptides were based on the hPop1 sequence, three on Rpp25, two on Rpp38 and one was an Rpp38 and hPop1 hybrid (Table 4). In a cluster analysis, Peptide 3 (ID Rpp38- ²²⁹ RELLDTSFEDLSKPK ²⁴³(SEQ ID NO: 25)), Peptide 6 (Rpp38- ²³³DTSFEDLSKPKRKLA²⁴⁷(SEQ ID NO: 102)) and Peptide 8 (Rpp38-²³³DTSFEDLSKPKRKLA²⁴⁷ / hPop1- ⁴¹⁸TGIIISDLTMEMNRF⁴³²(SEQ ID NO: 104)) clustered closest to SSc patients with nucleolar staining pattern (Fig. 3). Further comparative descriptive analysis showed significantly higher reactivity against peptide Rpp38- ²²⁹ RELLDTSFEDLSKPK ²⁴³(SEQ ID NO: 25) and Rpp38- ²³³ DTSFEDLSKPKRKLA ²⁴⁷ (SEQ ID NO: 102) in the SSc patients with nucleolar reactivity compared to the controls and in anti-Th/To antibody positive samples (measure by BIO-FLASH) compared to the controls. The major linear epitope of Rpp38 is shown both in a map and within the Rpp38 amino acid sequence (Fig. 4a and Fig 4b).

**Table 4: Designed Peptides**

| **No.** | **SEQ ID NO** | **Antigen** | **AA** | **Short ID** | **ID** |
|---|---|---|---|---|---|
| 1 | 98 | hPop1 | 15 | ⁴⁰⁰ hPop1 ⁴¹⁴ | hPopl- ⁴⁰⁰ IGDGTRDPCLPYSWI ⁴¹⁴ |
| 2 | 99 | Rpp25 | 20 | ⁵¹ Rpp25 ⁶⁸ | Rpp25- ⁵¹ AFATASMAQPATRAIVFSGC ⁶⁸ |
| 3 | 25 | Rpp38 | 15 | ²²⁹ Rpp38²⁴³ | Rpp38- ²²⁹ RELLDTSFEDLSKPK ²⁴³ |
| 4 | 100 | Rpp25 | 30 | ⁵⁴ Rpp25⁶⁸ / ¹²³ Rpp251³⁷ | Rpp25- ⁵⁴ TASMAQPATRAIVFS⁶⁸ / Rpp25-¹²³ AASLSVLKNVPGLAI ¹³⁷ |
| 5 | 101 | Rpp25 | 26 | ¹⁸⁹Rpp25¹⁹⁹/ ¹²³Rpp25¹³⁷ | Rpp25- ¹⁸⁹ EPGVADEDQTA¹⁹⁹ / Rpp25-¹²³AASLSVLKNVPGLAI¹³⁷ |
| 6 | 102 | Rpp38 | 15 | ²³³ Rpp38 ²⁴⁷ | Rpp38- ²³³ DTSFEDLSKPKRKLA ²⁴⁷ |
| 7 | 103 | hPop1 | 23 | ⁴¹⁸hPop1 ⁴⁴⁰ | hPop1- ⁴¹⁸ TGIIISDLTMEMNRFRLIGPLSH 440 |
| 8 | 104 | Rpp38 | 30 | ²³³Rpp38²⁴⁷/- ⁴¹⁸hPop1⁴³² | Rpp38- ²³³ DTSFEDLSKPKRKLA ²⁴⁷ /hPop1-⁴¹⁸TGIIISDLTMEMNRF ⁴³² |

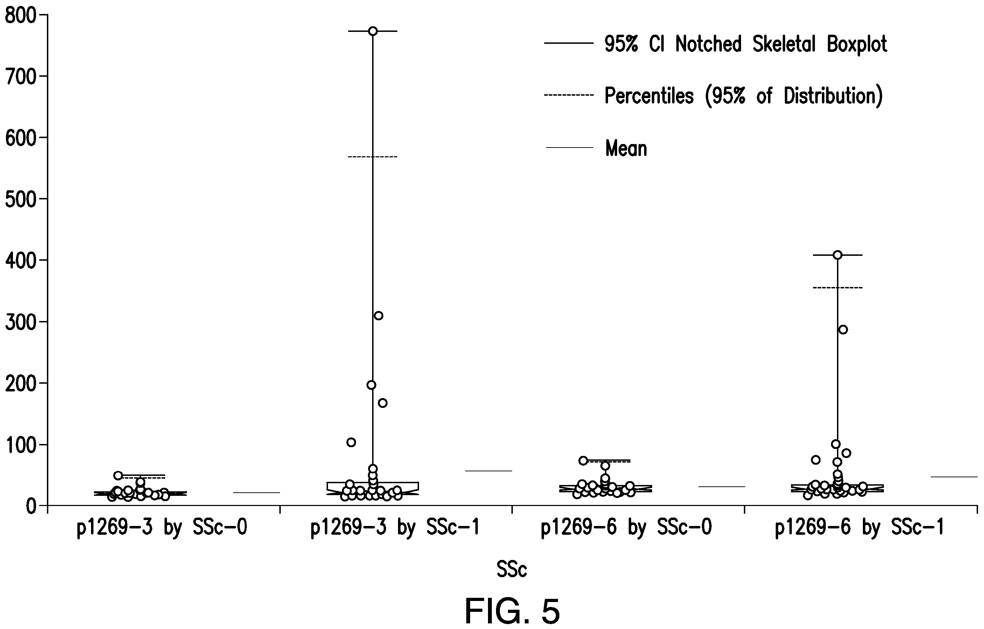

Reactivity to the synthetic peptides were tested in both SSc samples with nucleolar staining pattern and disease controls (SLE sample). As shown in Figure 5, reactivity to Peptide 3 and Peptide 6 are clearly higher in SSc samples compared with disease control.

### Correlation between anti-Rpp25 and anti-Rpp38 peptide antibodies

**Recombinant Rpp25 antigen and anti-Rpp25 immunoassay:** Recombinant full-length, his-tagged Rpp25 was generated and purified as previously described and used for Enzyme Linked Immunosorvent Assay ("ELISA") and Chemiluminescenece Assay ("CLIA") (Mahler M et al, Arthritis Res Ther 15:R50(2013)). The QUANTA Flash Rpp25 (research use only, INOVA Diagnostics Inc., San Diego, CA, USA) assay is a novel CLIA that is currently used for research purposes only and utilizes the BIO-FLASH^{®} instrument (Biokit s.a., Barcelona, Spain), fitted with a luminometer, as well as all the hardware and liquid handling accessories necessary to fully automate the assay. The QUANTA Flash assay for this study was developed using full-length, purified, recombinant human Rpp25 antigen coated onto paramagnetic beads. The principle of the QUANTA Flash Rpp25 assay performed on the BIO-FLASH instrument has recently been described(Mahler M et al, Arthritis Res Ther 15:R50(2013); Mahler M et al, Clin Chim Acta 413:719-26(2012)).

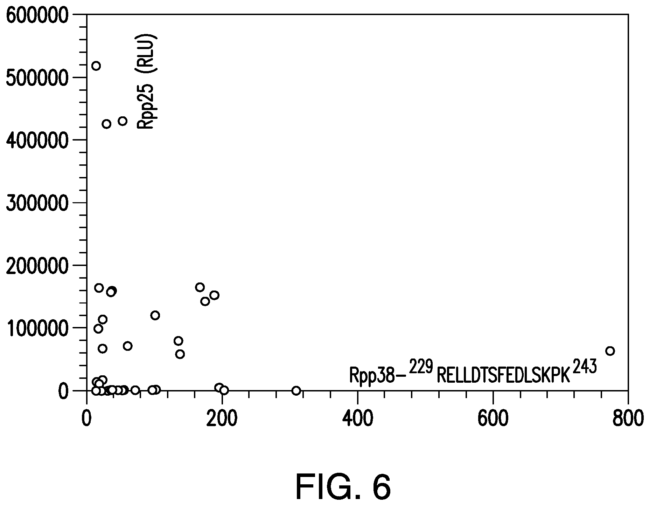

**Results:** Antibodies to the novel Rpp38 peptide showed significant correlation to antibodies to Rpp25 as measured by BIO-FLASH. Using 157 samples, the quantitative agreement according to the Spearman equation was 0.48 (95% CI 0.35 - 0.59; *p*<0.0001). However, the serum from subsets of patients reacted strongly with Rpp25 or with the novel Rpp38 peptide. (Fig. 6).

### Use of anti-Th/To test in the diagnosis of SSc and other diseases

Antinuclear antibodies are present in >90% of the patients and represent valuable biomarkers in the diagnosis of SSc (Mahler M et al, Ann N Y Acad Sci 1183:267-87 (2010); Satoh M et al, Mod Rheumatol 19:219-28 (2009)). However, since the ANA HEp-2 is by no means specific for a particular SARD (Mahler M et al, Clin Dev Immunol 2012:494356(2012); Satoh M et al, Arthritis Rheum 64:2319-27(2012)). Confirmatory testing with molecular targets is mandatory to confirm positive ANA results and to more accurately characterize SSc patients. Besides anti-centromere, anti-Scl-70 and anti-RNA Pol III antibodies as the major markers, antibodies to the Th/To antigens have been described in SSc patients. Almost all protein subunits of the RNase MRP and the evolutionarily related RNase P complex have been reported to be the target of anti-Th/To antibodies in SARD patients (Van Eenennaam H et al, Arthritis Rheum 46:3266-72(2002); Kuwana M et al, Ann Rheum Dis 61:842-6(2002)). Recent studies, using ELISA and CLIA, confirmed that Rpp25 is a major autoantigen targeted by anti-Th/To antibodies (Van Eenennaam H et al, Arthritis Rheum 46:3266-72(2002); Mahler M et al, Arthritis Res Ther 15:R50(2013)), being detected in approximately 60-100% of anti-Th/To antibody positive patients.

Although anti-Th/To antibodies are uncommon in serum samples from SARD patients, the observation that anti-Th/To antibodies are mostly detectable in SSc makes this specificity an important serological adjunct in the diagnosis of SSc. In addition, a high prevalence of anti-Th/To antibodies was found in ANA positive/ENA negative patients (Mahler M et al, J Rheumatol. 2014 Jun 15. [Epub ahead of print]). The anti-Th/To test can also have applications to non-SSc patients such as ILD since anti-Th/To antibodies have been reported in ~50% of anti-nucleolar antibody-positive idiopathic pulmonary fibrosis patients (Fischer A et al, J Rheumatol 33:1600-5 (2006)).

When the prevalence of anti-Th/To (hPop1) or anti-Rpp25 antibodies in SSc patients was analyzed, similar prevalences were found: 3.3% (hPop1)( Villalta D et al, Autoimmun Rev 12:114-20 (2012)), 2.1% (hPop1) (Bonroy C et al, J Immunol Methods 379:53-60(2012)) and 2.9% (Rpp25) (Mahler M et al, Arthritis Res Ther 15:R50(2013)). More importantly, the prevalence of anti-Th/To (by IP) and anti-Rpp25 antibodies (by ELISA) were very similar measured in the same patient cohort (Mahler M et al, Arthritis Res Ther 15:R50(2013); Krzyszczak ME et al, Clin Rheumatol 30:1333-9 (2011)).

Although a commercially available LIA contains a Th/To antigen, namely hPop1 as the analyte, a significant number (n=18) of anti-Th/To antibodies (identified by IP) were missed in a large cohort of Canadian SSc patients. This can be due to low prevalence of anti-hPop1 antibodies among anti-Th/To positive patients in this cohort or lack of reactivity with the hPop1 antigen used in LIA. In a study by Kuwana et al, Ann Rheum Dis 61:842-6(2002), anti-hPop1 antibodies were significantly more prevalent in anti-Th/To positive SSc patients, compared to anti-Th/To positive patients with other SARDs. In contrast, Rpp30 and Rpp38 were equally targeted by antibodies from SSc and non-SSc SARD patients. This is contradictory to our findings showing that reactivity of the SLE serum pool to linear peptides of hPop1 was higher compared to Rpp25 and Rpp38.
Although known for over 20 years, the clinical association of anti-Th/To antibodies is not fully established. Previous studies are mostly consistent in showing its association with lcSSc, however, association with more specific clinical features are somewhat inconsistent. Small numbers of anti-Th/To positive patients, differences in ethnicity and environment, differences in the detection methods, recruitment bias and others could explain the inconsistencies (Ceribelli A et al, J Rheumatol 37:2071-5(2010); Van Eenennaam H et al, Clin Exp Immunol 130:532-40(2002); Krzyszczak ME et al, Clin Rheumatol 30:1333-9 (2011); Mitri GM et al, Arthritis Rheum 48:203-9(2003); Walker JG et al, Curr Opin Rheumatol 19:580-91(2007)). Anti-Th/To antibodies have also been associated with pericarditis, ILD and have a high frequency of "intrinsic" pulmonary hypertension (Ceribelli A et al, J Rheumatol 37:2071-5(2010); Graf SW et al, Int J Rheum Dis;15:102-9(2012)). Compared with the anti-CENP positive patients, anti-Th/To lcSSc patients have more subtle cutaneous, vascular, and gastrointestinal involvement, but more often have certain features seen in diffuse SSc, such as pulmonary fibrosis and SSc renal crisis, as well as reduced survival compared to anti-CENP positive patients (Mitri GM et al, Arthritis Rheum 48:203-9(2003)). Like other SSc related autoantibodies, in patients with Raynaud's phenomenon anti-Th/To antibodies are risk factors that are predictive of emerging SSc (Koenig M et al, Arthritis Rheum 58:3902-12(2008)). Anti-Th/To positive patients demonstrated earlier development of nailfold capillary microscopy abnormalities than anti-CENP positive patients (Koenig M et al, Arthritis Rheum 58:3902-12(2008)). Anti-Th/To positive patients were reported to be younger and more frequently male compared to anti-CENP positive patients (Ceribelli A et al, J Rheumatol 37:2071-5(2010)). It has been reported that the prevalence of anti-Th/To antibodies might be higher in Caucasian Americans compared to African and Latin Americans (Krzyszczak ME et al, Clin Rheumatol 30:1333-9 (2011)).

In our cohort of SSc patients preselected by autoantibody reactivity, we confirmed associations of anti-Th/To (complex) and anti-Rpp25 antibodies with ILD and abnormal nailfold capillaroscopy.

Despite the low prevalence of anti-Th/To antibodies, testing for those antibodies and the sub-specificities (anti-Rpp25, anti-Rpp38 and anti-hPop1 antibodies) can have significant value for patient stratification (Steen VD, Semin Arthritis Rheum 35:35-42(2005); Walker UA et al, Ann Rheum Dis 66:754-63(2007)). In a previous study dcSSc and lcSSc subsets were associated with particular organ manifestations, but in the report by Walker et al the clinical distinction appeared superseded by an antibody based classification in predicting some SSc related complications (Walker UA et al, Ann Rheum Dis 66:754-63(2007)).

## Claims

1. A purified peptide consisting of the amino acid sequence of SEQ ID NO: 25.

2. A complex comprising a purified peptide of claim 1, and an anti-Th/To antibody, wherein the complex is optionally in solution; or immobilized on a surface.

3. A method for detecting an anti-Th/To antibody in a subject comprising:
(a) contacting a sample from the subject with the purified peptide of claim 1 to form a complex between an anti-Th/To antibody and the purified peptide; and
(b) detecting the presence or absence of the anti-Th/To antibody-purified peptide complex in the sample,
wherein said anti-Th/To antibody comprises an anti-Rpp25 antibody, an anti-Rpp38 antibody, an anti-hPopl antibody, or any combination thereof; and
wherein the method optionally further comprises:
(c) an initial step of preparing the purified peptide; or
(d) immobilizing the purified peptide of a surface.

4. The method of claim 3, wherein:
(i) the presence or absence of the anti-Th/To antibody-purified peptide complex is detected by an assay selected from the group consisting of an enzyme-linked immunosorbent assay (ELISA), a fluorescent immunosorbent assay (FIA), a chemiluminescent immunosorbent assay (CLIA), a radioimmunoassay (RIA), an enzyme multiplied immunoassay, a solid phase radioimmunoassay (SPROA), a fluorescence polarization (FP) assay, a fluorescence resonance energy transfer (FRET) assay, a time-resolved fluorescence resonance energy transfer (TR-FRET) assay and a surface plasmon resonance (SPR) assay; or
(ii) detecting the presence or absence of the anti-Th/To antibody-purified peptide complex comprises establishing a level of the anti-Th/To antibody in the sample, or comparing the level of anti-Th/To antibody in the sample from the subject to a control level of anti-Th/To antibody in a sample from a healthy control individual, wherein an increase in the anti-Th/To antibody level in the sample compared to the control level indicates that the subject has systemic sclerosis (SSc), wherein said SSc is optionally limited cutaneous systemic sclerosis (lcSSc).

5. The method of claim 3, wherein the subject:
(i) is suspected of having systemic sclerosis (SSc), rheumatoid arthritis (RA), pericarditis or interstitial lung disease (ILD);
(ii) has a negative ANA result;
(iii) is negative for at least one autoantibody selected from the group consisting of an anti-topoisomerase I (topo-I) antibody, an anti-centromere (CENP) antibody and an anti-RNA polymerase III (RNAP) antibody;
(iv) is negative for an anti-PM/Sc1 complex (exosome) antibody or an anti-U3RNP/fibrillarin antibody; or
(v) is suspected of having limited cutaneous systemic sclerosis (lcSSc).

6. A method of diagnosing systemic sclerosis (SSc) or limited cutaneous systemic sclerosis (lcSSc) in a human subject suspected of having SSc, comprising:
(a) contacting a sample from the subject with the purified peptide of claim 1 to form a complex between an anti-Th/To antibody and the purified peptide; and
(b) detecting the presence or absence of the anti-Th/To antibody-purified peptide complex in the sample,
wherein the presence of the anti-Th/To antibody-purified peptide complex in the sample indicates that the subject has SSc; and
wherein said anti-Th/To antibody comprises an anti-Rpp25 antibody, an anti-Rpp38 antibody, an anti-hPopl antibody, or any combination thereof.

7. The method of claim 6, wherein the human subject:
(i) has a negative ANA result;
(ii) is negative for at least one autoantibody selected from the group consisting of an anti-topoisomerase I (topo-I) antibody, an anti-centromere (CENP) antibody and an anti-RNA polymerase III (RNAP) antibody; or
(iii) is negative for an anti-PM/Sc1 complex (exosome) antibody or an anti-U3RNP/fibrillarin antibody.

8. A method of determining the prognosis of systemic sclerosis (SSc) or limited cutaneous systemic sclerosis (lcSSc) in a human subject, comprising:
(a) contacting a sample from the subject with the purified peptide of claim 1 to form a complex between an anti-Th/To antibody and the purified peptide; and
(b) detecting the presence or absence of the anti-Th/To antibody-purified peptide complex in the sample,
wherein the presence of the anti-Th/To antibody-purified peptide complex in the sample indicates the course of SSc progression in the human subject; and
wherein said anti-Th/To antibody comprises an anti-Rpp25 antibody, an anti-Rpp38 antibody, an anti-hPopl antibody, or any combination thereof.

9. The method of claim 8, wherein:
(i) the presence of the anti-Th/To antibody-purified peptide complex in the sample indicates that the subject is at a greater risk of developing SSc than the absence of the anti-Th/To antibody-purified peptide complex;
(ii) detecting the presence or absence of the anti-Th/To antibody-purified peptide complex in the sample comprises determining the level of anti-Th/To antibodies in the sample; or
(iii) detecting the presence or absence of the anti-Th/To antibody-purified peptide complex in the sample comprises determining the level of anti-Th/To antibodies in the sample, wherein a higher level of anti-Th/To antibodies in the sample indicate a higher risk that an asymptomatic subject will develop SSc than a lower level of anti-Th/To antibodies.

10. The method of claim 8, wherein the human subject is:
(i) an asymptomatic subject suspected to be at risk of developing SSc; or
(ii) a SSc patient having a clinical symptom of SSc.

11. A method of monitoring the efficacy of a systemic sclerosis (SSc) or limited cutaneous systemic sclerosis (lcSSc) treatment in a SSc or IcSSc patient, comprising:
(a) contacting two or more samples obtained from the patient at a first and at least one subsequent time point during SSc treatment with the purified peptide of claim 1 to form a complex between an anti-Th/To antibody and the purified peptide;
(b) determining the levels of the anti-Th/To antibody in the two or more samples; and
(c) comparing the levels of anti-Th/To antibodies in the two or more samples,
wherein said anti-Th/To antibody comprise an anti-Rpp25 antibody, an anti-Rpp38 antibody, an anti-hPopl antibody, or any combination thereof; and
wherein a decreased level of the anti-Th/To antibody in a sample obtained at a subsequent time point relative to a level of the anti-Th/To antibody in a sample obtained at the first time point indicates that the SSc treatment is efficacious;
wherein optionally the level of anti-Th/To antibody in the samples obtained at the first time point are decreased by more than 10%, more than 20%, more than 30%, more than 40%, more than 50%, more than 60%, more than 70%, more than 80%, more than 90%, more than 95%, or more than 99% in the subsequence time point.

12. A kit for detecting an anti-Th/To antibody in a sample from a subject or for diagnosing an autoimmune disease, preferably systemic sclerosis (SSc), rheumatoid arthritis (RA), pericarditis or interstitial lung disease (ILD), comprising the purified peptide of claim 1 and an ancillary reagent,
wherein said anti-Th/To antibody comprises an anti-Rpp25 antibody, an anti-Rpp38 antibody, an anti-hPopl antibody, or any combination thereof;
wherein optionally:
(i) the ancillary reagent comprises one or more ancillary reagents selected from the group consisting of:
(a) a secondary antibody, preferably selected from an anti-human IgA antibody, anti-human IgD antibody, anti-human IgE antibody, antihuman IgG antibody, and anti-human IgM antibody;
(b) a detection reagent, preferably a fluorescent detection reagent or a luminescent detection reagent, wherein the luminescent detection reagent preferably comprises luminol or luciferin;
(c) an immobilization buffer,
(d) a blocking buffer,
(e) a washing buffer, and
(f) a detection buffer;
or
(ii) the kit further comprises:
(a) a microtiter plate having wells, wherein the microtiter plate is preferably a 96-well plate, a 384-well plate, or a 1536-well plate, or wherein the purified peptide is optionally immobilized in one or more wells of the microtiter plate;
(b) instruction for using the subunits of the kit for detecting an anti-Th/To antibody in the sample from the subject or for diagnosing the autoimmune disease;
(c) a packaging having a label indicating that the kit is used for diagnosis, prognosis or monitoring of SSc, RA, ILD or IcSSc, wherein optionally said label is FDA-approved;
(d) a packaging having a label indicating that the kit is used as an In Vitro Diagnsitc (IVD) companion diagnostic device, wherein optionally said label is FDA-approved; or
(e) a packaging having a label indicating that the kit is used with a systemic sclerosis (SSc) drug, wherein optionally said label is FDA-approved.

## Patentansprüche

1. Aufgereinigtes Peptid, bestehend aus der Aminosäuresequenz nach SEQ ID NO: 25.

2. Komplex, umfassend ein aufgereinigtes Peptid nach Anspruch 1 und einen anti-Th/To-Antikörper, wobei der Komplex optional in Lösung vorliegt oder auf einer Oberfläche immobilisiert ist.

3. Verfahren zum Nachweis eines anti-Th/To-Antikörpers in einem Subjekt, umfassend:
(a) Inkontaktbringen einer Probe von dem Subjekt mit dem aufgereinigten Peptid nach Anspruch 1, um einen Komplex zwischen einem anti-Th/To-Antikörper und dem aufgereinigten Peptid zu bilden; und
(b) Nachweisen der An- oder Abwesenheit des Komplexes aus anti-Th/To-Antikörper und aufgereinigtem Peptid in der Probe,
wobei der anti-Th/To-Antikörper einen anti-Rpp25-Antikörper, einen anti-Rpp38-Antikörper, einen anti-hPopl-Antikörper oder eine beliebige Kombination davon umfasst; und
wobei das Verfahren optional weiter umfasst:
(c) einen ersten Schritt der Herstellung des aufgereinigten Peptids; oder
(d) die Immobilisierung des aufgereinigten Peptids auf einer Oberfläche.

4. Verfahren nach Anspruch 3, wobei:
(i) die An- oder Abwesenheit des Komplexes aus anti-Th/To-Antikörper und aufgereinigtem Peptid durch einen Assay nachgewiesen wird, ausgewählt aus der Gruppe, bestehend aus einem Enzym-Immunoassay (ELISA), einem Fluoreszenz-Immunoassay (FIA), einem Chemilumineszenz-Immunoassay (CLIA), einem Radioimmunoassay (RIA), einem enzymverstärkten Immunoassay, einem Festphasen-Radioimmunoassay (SPROA), einem Fluoreszenzpolarisationsassay (FP), einem Fluoreszenzresonanzenergietransferassay (FRET), einem zeitaufgelösten Fluoreszenzresonanzenergietransferassay (TR-FRET) und einem Oberflächenplasmonresonanzassay (SPR); oder
(ii) das Nachweisen der An- oder Abwesenheit des Komplexes aus anti-Th/To-Antikörper und aufgereinigtem Peptid das Bestimmen eines Spiegels des anti-Th/To-Antikörpers in der Probe oder das Vergleichen des Spiegels des anti-Th/To-Antikörpers in der Probe des Probanden mit einem Kontrollspiegel des anti-Th/To-Antikörpers in einer Probe von einem gesunden Kontrollindividuum umfasst, wobei ein Anstieg des anti-Th/To-Antikörperspiegels in der Probe im Vergleich zum Kontrollspiegel darauf hinweist, dass das Subjekt systemische Sklerose (SSc) aufweist, wobei die SSc optional limitierte kutane systemische Sklerose (IcSSc) ist.

5. Verfahren nach Anspruch 3, wobei das Subjekt:
(i) im Verdacht steht, systemische Sklerose (SSc), rheumatoide Arthritis (RA), Perikarditis oder interstitielle Lungenerkrankung (ILD) aufzuweisen;
(ii) ein negatives ANA-Ergebnis aufweist;
(iii) negativ für mindestens einen Autoantikörper ist, ausgewählt aus der Gruppe, bestehend aus einem anti-Topoisomerase-I-Antikörper (topo-I), einem anti-Zentromer-Antikörper (CENP) und einem anti-RNA-Polymerase-III-Antikörper (RNAP);
(iv) negativ für einen anti-PM/Sc1-Komplex (Exosom)-Antikörper oder einen anti-U3RNP/Fibrillarin-Antikörper ist; oder
(v) im Verdacht steht, eine limitierte kutane systemische Sklerose (IcSSc) aufzuweisen.

6. Verfahren zur Diagnose von systemischer Sklerose (SSc) oder limitierter kutaner systemischer Sklerose (IcSSc) bei einem menschlichen Subjekt, das im Verdacht steht, SSc aufzuweisen, umfassend:
(a) Inkontaktbringen einer Probe des Subjekts mit dem aufgereinigten Peptid nach Anspruch 1, um einen Komplex zwischen einem anti-Th/To-Antikörper und dem aufgereinigten Peptid zu bilden; und
(b) Nachweisen der An- oder Abwesenheit des Komplexes aus anti-Th/To-Antikörper und aufgereinigtem Peptid in der Probe,
wobei die Anwesenheit des Komplexes aus anti-Th/To-Antikörper und aufgereinigtem Peptid in der Probe anzeigt, dass das Subjekt SSc aufweist; und
wobei der anti-Th/To-Antikörper einen anti-Rpp25-Antikörper, einen anti-Rpp38-Antikörper, einen anti-hPopl-Antikörper oder eine beliebige Kombination davon umfasst.

7. Verfahren nach Anspruch 6, wobei das menschliche Subjekt:
(i) ein negatives ANA-Ergebnis aufweist;
(ii) negativ für mindestens einen Autoantikörper ist, ausgewählt aus der Gruppe, bestehend aus einem anti-Topoisomerase-I-Antikörper (topo-I), einem anti-Zentromer-Antikörper (CENP) und einem anti-RNA-Polymerase-III-Antikörper (RNAP);
(iii) negativ für einen anti-PM/Sc1-Komplex (Exosom)-Antikörper oder einen anti-U3RNP/Fibrillarin-Antikörper ist.

8. Verfahren zur Bestimmung der Prognose von systemischer Sklerose (SSc) oder limitierter kutaner systemischer Sklerose (IcSSc) bei einem menschlichen Subjekt, umfassend:
(a) Inkontaktbringen einer Probe von dem Subjekt mit dem aufgereinigten Peptid nach Anspruch 1, um einen Komplex zwischen einem anti-Th/To-Antikörper und dem aufgereinigten Peptid zu bilden; und
(b) Nachweisen der An- oder Abwesenheit des Komplexes aus anti-Th/To-Antikörper und aufgereinigtem Peptid in der Probe,
wobei die Anwesenheit des Komplexes aus anti-Th/To-Antikörper und aufgereinigtem Peptid in der Probe den Verlauf der SSc-Progression bei dem menschlichen Subjekt anzeigt; und
wobei der anti-Th/To-Antikörper einen anti-Rpp25-Antikörper, einen anti-Rpp38-Antikörper, einen anti-hPopl-Antikörper oder eine beliebige Kombination davon umfasst.

9. Verfahren nach Anspruch 8, wobei:
(i) die Anwesenheit des Komplexes aus anti-Th/To-Antikörper und aufgereinigtem Peptid in der Probe anzeigt, dass das Subjekt ein höheres Risiko aufweist, an SSc zu erkranken, als die Abwesenheit des Komplexes aus anti-Th/To-Antikörper und aufgereinigtem Peptid;
(ii) das Nachweisen der An- oder Abwesenheit des Komplexes aus anti-Th/To-Antikörper und aufgereinigtem Peptid das Bestimmen eines Spiegels des anti-Th/To-Antikörpers in der Probe umfasst; oder
(iii) das Nachweisen der An- oder Abwesenheit des Komplexes aus anti-Th/To-Antikörper und aufgereinigtem Peptid das Bestimmen eines Spiegels des anti-Th/To-Antikörpers in der Probe umfasst, wobei ein höherer Spiegel an anti-Th/To-Antikörpern in der Probe ein höheres Risiko anzeigt, dass ein asymptomatisches Subjekt SSc entwickelt, als ein niedrigerer Spiegel an anti-Th/To-Antikörpern.

10. Verfahren nach Anspruch 8, wobei das menschliche Subjekt:
(i) eine asymptomatisches Subjekt ist, das im Verdacht steht, ein Risiko für die Entwicklung von SSc aufzuweisen; oder
(ii) ein SSc-Patient ist, der ein klinisches Symptom von SSc aufweist.

11. Verfahren zur Überwachung der Wirksamkeit einer Behandlung von systemischer Sklerose (SSc) oder limitierter kutaner systemischer Sklerose (IcSSc) bei einem SSc- oder IcSSc-Patienten, umfassend:
(a) Inkontaktbringen von zwei oder mehr Proben, die zu einem ersten und mindestens einem nachfolgenden Zeitpunkt während der SSc-Behandlung von dem Patienten erhalten wurden, mit dem aufgereinigten Peptid nach Anspruch 1, um einen Komplex zwischen einem anti-Th/To-Antikörper und dem aufgereinigten Peptid zu bilden;
(b) Bestimmen des Spiegels des anti-Th/To-Antikörpers in den zwei oder mehr Proben; und
(c) Vergleichen der Spiegel der anti-Th/To-Antikörper in den zwei oder mehr Proben,
wobei der anti-Th/To-Antikörper einen anti-Rpp25-Antikörper, einen anti-Rpp38-Antikörper, einen anti-hPopl-Antikörper oder eine beliebige Kombination davon umfasst; und
wobei ein verringerter Spiegel des anti-Th/To-Antikörpers in einer Probe, die zu einem späteren Zeitpunkt erhalten wurde, im Vergleich zu einem Spiegel des anti-Th/To-Antikörpers in einer Probe, die zum ersten Zeitpunkt erhalten wurde, anzeigt, dass die SSc-Behandlung wirksam ist;
wobei optional der Spiegel des anti-Th/ To-Antikörpers in den Proben, die zum ersten Zeitpunkt erhalten wurden, um mehr als 10%, mehr als 20%, mehr als 30%, mehr als 40%, mehr als 50%, mehr als 60%, mehr als 70%, mehr als 80%, mehr als 90%, mehr als 95% oder mehr als 99% verringert ist.

12. Kit zum Nachweis eines anti-Th/To-Antikörpers in einer Probe von einem Subjekt oder zur Diagnose einer Autoimmunerkrankung, vorzugsweise systemischer Sklerose (SSc), rheumatoider Arthritis (RA), Perikarditis oder interstitieller Lungenerkrankung (ILD), umfassend das aufgereinigte Peptid nach Anspruch 1 und ein Hilfsreagenz,
wobei der anti-Th/To-Antikörper einen anti-Rpp25-Antikörper, einen anti-Rpp38-Antikörper, einen anti-hPopl-Antikörper oder eine beliebige Kombination davon umfasst;
wobei optional:
(i) das Hilfsreagenz ein oder mehrere Hilfsreagenzien umfasst, ausgewählt aus der Gruppe, bestehend aus:
(a) einem sekundären Antikörper, vorzugsweise ausgewählt aus einem anti-human-IgA-Antikörper, einem anti-human-IgD-Antikörper, einem anti-human-IgE-Antikörper, einem anti-human-IgG-Antikörper und einem anti-human-IgM-Antikörper;
(b) ein Nachweisreagenz, vorzugsweise ein fluoreszierendes Nachweisreagenz oder ein lumineszierendes Nachweisreagenz, wobei das lumineszierende Nachweisreagenz vorzugsweise Luminol oder Luciferin umfasst;
(c) einen Immobilisierungspuffer,
(d) einen Blockierungspuffer,
(e) einen Waschpuffer und
(f) einen Nachweispuffer;
oder
(ii) das Kit weiter umfasst:
(a) eine Mikrotiterplatte mit Vertiefungen, wobei die Mikrotiterplatte vorzugsweise eine 96-Well-Platte, eine 384-Well-Platte oder eine 1536-Well-Platte ist, oder wobei das aufgereinigte Peptid optional in einer oder mehreren Vertiefungen der Mikrotiterplatte immobilisiert ist;
(b) eine Anleitung zur Verwendung der Untereinheiten des Kits zum Nachweis eines anti-Th/To-Antikörpers in der Probe von dem Subjekt oder zur Diagnose der Autoimmunerkrankung;
(c) eine Verpackung mit einem Etikett, das angibt, dass das Kit zur Diagnose, Prognose oder Überwachung von SSc, RA, ILD oder IcSSc verwendet wird, wobei das Etikett optional von der FDA zugelassen ist;
(d) eine Verpackung mit einem Etikett, das angibt, dass das Kit als begleitende diagnostische Vorrichtung für die In-Vitro-Diagnostik (IVD) verwendet wird, wobei das Etikett optional von der FDA zugelassen ist; oder
(e) eine Verpackung mit einem Etikett, das angibt, dass das Kit zusammen mit einem Medikament gegen systemische Sklerose (SSc) verwendet wird, wobei das Etikett optional von der FDA zugelassen ist.

## Revendications

1. Peptide purifié consistant en la séquence des acides aminés de SEQ ID N°25.

2. Complexe comprenant un peptide purifié selon la revendication 1, et un anticorps anti-Th/To, dans lequel le complexe peut être optionnellement en solution, ou immobilisé sur une surface.

3. Procédé de détection d'un anticorps anti-Th/To chez un individu, comprenant :
a) la mise en contact d'un échantillon de l'individu avec le peptide purifié selon la revendication 1, pour former un complexe entre un anticorps anti-Th/To et le peptide purifié, et
b) la détection de la présence ou de l'absence du complexe anticorps anti-Th/To-peptide purifié dans l'échantillon,
dans lequel ledit anticorps anti-Th/To comprend un anticorps anti-Rpp25, un anticorps anti-Rpp38, un anticorps anti-hPopl, ou une combinaison de ceux-ci, et dans lequel le procédé comprend en outre, le cas échéant :
c) une étape initiale de préparation du peptide purifié, ou
d) l'immobilisation du peptide purifié sur une surface.

4. Procédé selon la revendication 3, dans lequel
(i) la présence ou l'absence du complexe anticorps anti-Th/To-peptide purifié est détectée par un dosage choisi parmi le groupe consistant en un dosage immunoenzymatique (ELISA), un immunodosage fluorescent (FIA), un immunodosage par chimioluminescence (CLIA), un radioimmunodosage (RIA), un immunodosage enzymatique multiplié, un radioimmunodosage en phase solide (SPROA), un dosage de polarisation de fluorescence (FP), un dosage par transfert d'énergie de fluorescence par résonance (FRET), un dosage par transfert d'énergie de fluorescence par résonance avec résolution temporelle (RT-FRET), et un dosage de résonance de plasmons de surface (SPR) ; ou
(ii) la détection de la présence ou de l'absence du complexe anticorps anti-Th/To-peptide purifié comprend la détermination du taux d'anticorps anti-Th/To dans l'échantillon, ou la comparaison du taux d'anticorps anti-Th/To dans l'échantillon de l'individu avec un taux d'anticorps anti-Th/To de contrôle dans un échantillon d'un individu en bonne santé, où une augmentation du taux d'anticorps anti-Th/To dans l'échantillon par rapport au taux de contrôle indique que l'individu présente une sclérose systémique (ScS), où la ScS est le cas échéant, une sclérose systémique cutanée limitée (ScScl).

5. Procédé selon la revendication 3, dans lequel l'individu :
(i) est suspecté de souffrir d'une sclérose systémique (ScS), d'une polyarthrite rhumatoïde (PR), d'une péricardite ou d'une pneumopathie interstitielle diffuse (PID) ;
(ii) présente un résultat ANA négatif ;
(iii) est négatif pour au moins l'un des autoanticorps choisis parmi le groupe consistant en un anticorps anti-topoisomérase I (topo-I), un anticorps anti-centromère (CENP) et un anticorps anti ARN polymérase III (RNAP) ;
(iv) est négatif pour un anticorps anti- complexe PM/Sc1 (exosome) ou un anticorps anti-U3RNP/fibrillarine, ou
(v) est suspecté de présenter une sclérose systémique cutanée limitée (ScScl).

6. Procédé de diagnostic d'une sclérose systémique (ScS) ou d'une sclérose systémique cutanée limitée (ScScl) chez un individu humain suspecté de souffrir d'une ScS, comprenant :
a) la mise en contact d'un échantillon de l'individu avec le peptide purifié selon la revendication 1, pour former un complexe entre un anticorps anti-Th/To et le peptide purifié, et
b) la détection de la présence ou de l'absence du complexe anticorps anti-Th/To-peptide purifié dans l'échantillon,
dans lequel la présence du complexe anticorps anti-Th/To-peptide purifié dans l'échantillon indique que l'individu est atteint d'une ScS, et
dans lequel ledit anticorps anti-Th/To comprend un anticorps anti-Rpp25, un anticorps anti-Rpp38, un anticorps anti-hPopl, ou une combinaison de ceux-ci.

7. Procédé selon la revendication 6, dans lequel l'individu humain :
(i) présente un résultat ANA négatif ;
(ii) est négatif pour au moins l'un des autoanticorps choisis parmi le groupe consistant en un anticorps anti-topoisomérase I (topo-I), un anticorps anti-centromère (CENP) et un anticorps anti ARN polymérase III (RNAP) ; ou
(iii) est négatif pour un anticorps anti- complexe PM/Sc1 (exosome) ou un anticorps anti-U3RNP/fibrillarine.

8. Procédé de détermination du pronostic de sclérose systémique (ScS) ou de sclérose systémique cutanée limitée (ScScl) chez un individu humain, comprenant :
a) la mise en contact d'un échantillon de l'individu avec le peptide purifié selon la revendication 1, pour former un complexe entre un anticorps anti-Th/To et le peptide purifié, et
b) la détection de la présence ou de l'absence du complexe anticorps anti-Th/To-peptide purifié dans l'échantillon,
dans lequel la présence du complexe anticorps anti-Th/To-peptide purifié dans l'échantillon indique le processus de progression de la ScS chez l'individu humain, et
dans lequel ledit anticorps anti-Th/To comprend un anticorps anti-Rpp25, un anticorps anti-Rpp38, un anticorps anti-hPopl, ou une combinaison de ceux-ci.

9. Procédé selon la revendication 8, dans lequel :
(i) la présence du complexe anticorps anti-Th/To-peptide purifié dans l'échantillon indique que l'individu a un plus grand risque de développer une ScS que l'absence du complexe anticorps anti-Th/To-peptide purifié ;
(ii) la détection de la présence ou de l'absence du complexe anticorps anti-Th/To-peptide purifié dans l'échantillon comprend la détermination du taux d'anticorps anti-Th/To dans l'échantillon ; ou
(iii) la détection de la présence ou de l'absence du complexe anticorps anti-Th/To-peptide purifié dans l'échantillon comprend la détermination du taux d'anticorps anti-Th/To dans l'échantillon, où un taux plus élevé d'anticorps anti-Th/TO dans l'échantillon indique un risque plus élevé qu'un individu asymptomatique va développer une ScS par rapport à un taux plus faible d'anticorps anti-Th/To.

10. Procédé selon la revendication 8, dans lequel l'individu humain est :
(i) un individu asymptomatique suspecté de présenter un risque de développer une ScS, ou
(ii) un patient atteint de ScS ayant un symptôme clinique de ScS.

11. Procédé de surveillance de l'efficacité d'un traitement de la sclérose systémique (ScS) ou de la sclérose systémique cutanée limitée (ScScl) chez un patient atteint de ScS ou de ScScl, comprenant :
a) la mise en contact de deux échantillons ou plus obtenus auprès du patient à un premier moment et au moins un moment ultérieur pendant le traitement de la ScS, avec le peptide purifié selon la revendication 1, pour former un complexe entre un anticorps anti-Th/To et le peptide purifié, et
b) la détermination des taux de l'anticorps anti-Th/To dans les deux échantillons ou plus, et
c) la comparaison des taux d'anticorps anti-Th/TO dans les deux échantillons ou plus,
dans lequel ledit anticorps anti-Th/To comprend un anticorps anti-Rpp25, un anticorps anti-Rpp38, un anticorps anti-hPopl, ou une combinaison de ceux-ci, et
dans lequel une réduction du taux d'anticorps anti-Th/To dans un échantillon obtenu à un moment ultérieur, par rapport au taux d'anticorps anti-Th/To dans un échantillon obtenu au premier moment, indique que le traitement de la ScS est efficace ;
dans lequel, le cas échéant, le taux d'anticorps anti-Th/To dans les échantillons obtenus au premier moment est diminué de plus de 10 %, plus de 20 %, plus de 30 %, plus de 40 %, plus de 50 %, plus de 60 %, plus de 70 %, plus de 80 %, plus de 90 %, plus de 95 %, ou plus de 99 % dans l'échantillon du moment ultérieur.

12. Kit de détection d'un anticorps anti-Th/To dans un échantillon d'un individu ou pour la détection d'une maladie auto-immune, de préférence la sclérose systémique (ScS), la polyarthrite rhumatoïde (PR), la péricardite ou la pneumopathie interstitielle diffuse (PID), comprenant le peptide purifié selon la revendication 1, et un réactif auxiliaire, dans lequel ledit anticorps anti-Th/To comprend un anticorps anti-Rpp25, un anticorps anti-Rpp38, un anticorps anti-hPopl, ou une combinaison de ceux-ci ;
dans lequel, le cas échéant :
(i) le réactif auxiliaire comprend un ou plusieurs réactifs auxiliaires choisis parmi le groupe consistant en :
(a) un deuxième anticorps, de préférence choisi parmi un anticorps anti IgA - humain, un anticorps anti IgD -humain, un anticorps anti IgE -humain, un anticorps anti IgG -humain, et un anticorps anti IgM -humain ;
(b) un agent de détection, de préférence un agent de détection fluorescent ou un réactif de détection luminescent, dans lequel le réactif de détection luminescent comprend de préférence, le luminol ou la luciférine ;
(c) un tampon d'immobilisation ;
(d) un tampon de blocage ;
(e) un tampon de lavage, et
(f) un tampon de détection ;
ou
(ii) le kit comprend en outre :
(a) une plaque de microtitrage avec des puits, la plaque de microtitrage étant de préférence, une plaque à 96 puits, une plaque à 384 puits ou une plaque à 1536 puits, ou le peptide purifié étant le cas échéant, immobilisé dans un ou plusieurs puits de la plaque de microtitrage ;
(b) les instructions d'utilisation des sous-unités du kit pour la détection d'un anticorps anti-Th/To dans l'échantillon d'un individu ou pour le diagnostic d'une maladie auto-immune ;
(c) un conditionnement comprenant une notice mentionnant que le kit est utilisé pour le diagnostic, le pronostic ou la surveillance de la ScS, de la PR, de la PID ou de la ScScl, ladite notice pouvant optionnellement être approuvée par la FDA ;
(d) un conditionnement comportant une notice mentionnant que le kit est utilisé en tant que dispositif de diagnostic compagnon de diagnostic in vitro (DIV), ladite notice pouvant optionnellement être approuvée par la FDA ; ou
(e) un conditionnement comportant une notice mentionnant que le kit est utilisé avec un médicament pour la sclérose systémique (ScS), ladite notice pouvant optionnellement être approuvée par la FDA.
